(19) **Europäisches Patentamt / European Patent Office / Office européen des brevets**

(11) **EP 4 588 417 A1**

(12) **EUROPEAN PATENT APPLICATION**
published in accordance with Art. 153(4) EPC

(43) Date of publication:
**23.07.2025 Bulletin 2025/30**

(21) Application number: **23865374.5**

(22) Date of filing: **06.09.2023**

(51) International Patent Classification (IPC):
**A61B 1/00** (2006.01)     **G02B 5/04** (2006.01)

(52) Cooperative Patent Classification (CPC):
**A61B 1/00; G02B 5/04**

(86) International application number:
**PCT/JP2023/032474**

(87) International publication number:
**WO 2024/058019 (21.03.2024 Gazette 2024/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **16.09.2022 JP 2022148079**

(71) Applicant: **Sony Group Corporation
Tokyo 108-0075 (JP)**

(72) Inventors:
• **KUNIMATSU, Shinya
Tokyo 108-0075 (JP)**
• **OKI, Tomoyuki
Tokyo 108-0075 (JP)**

(74) Representative: **D Young & Co LLP
3 Noble Street
London EC2V 7BQ (GB)**

(54) **IMAGING DEVICE AND MEDICAL OBSERVATION SYSTEM**

(57)     An imaging device includes a prism that separates visible light and fluorescence included in light from a target to be observed, a visible-light imaging sensor that captures the visible light separated by the prism, and a fluorescence imaging sensor that captures the fluorescence separated by the prism, in which a ratio of a pixel spacing in the fluorescence imaging sensor to a pixel spacing in the visible-light imaging sensor is 1 or more and less than 5.

FIG.1

**Description**

Field

The present disclosure relates to an imaging device and a medical observation system.

Background

For example, Patent Literature 1 discloses a technology of separating visible light and fluorescence from a target to be observed to be captured with different imaging sensors.

Citation List

Patent Literature

Patent Literature 1: JP 2017-53890 A

Summary

Technical Problem

When the number of pixels of an imaging sensor is increased for high-resolution imaging, the light receiving area per pixel is narrowed by the number of pixels increased, and sensitivity may be insufficient. In particular, it is difficult to capture fluorescence clearly because fluorescence is weaker than visible light. There still remains need for study on a technology of achieving both capturing visible light with high resolution and capturing of fluorescence clearly.
One aspect of the present disclosure is to enable capturing visible light with high resolution and enable capturing fluorescence clearly.

Solution to Problem

An imaging device according to one aspect of the present disclosure includes: a prism that separates visible light and fluorescence included in light from a target to be observed; a visible-light imaging sensor that captures the visible light separated by the prism; and a fluorescence imaging sensor that captures the fluorescence separated by the prism, wherein a ratio of a pixel spacing in the fluorescence imaging sensor to a pixel spacing in the visible-light imaging sensor is 1 or more and less than 5.
A medical observation system according to one aspect of the present disclosure includes an imaging device that captures a surgical site, wherein the imaging device includes: a prism that separates visible light and fluorescence included in light from the surgical site; a visible-light imaging sensor that captures the visible light separated by the prism; and a fluorescence imaging sensor that captures the fluorescence separated by the prism, and a ratio of a pixel spacing in the fluorescence imaging sensor to a pixel spacing in the visible-light imaging sensor is 1 or more and less than 5.

Brief Description of Drawings

FIG. 1 is a diagram illustrating an example of a schematic configuration of an imaging device according to a first embodiment.
FIG. 2 is a graph illustrating an example of a characteristic of a dichroic film 65.
FIG. 3 is a diagram illustrating an example of a schematic configuration of a visible-light imaging sensor.
FIG. 4 is a diagram illustrating an example of a pixel spacing in the visible-light imaging sensor.
FIG. 5 is a diagram illustrating an example of a pixel spacing in the visible-light imaging sensor.
FIG. 6 is a graph illustrating an example of a characteristic of a long-pass filter.
FIG. 7 is a diagram illustrating an example of a schematic configuration of a fluorescence imaging sensor.
FIG. 8 is a diagram illustrating an example of a pixel spacing in the fluorescence imaging sensor.
FIG. 9 is a diagram illustrating an example of a pixel spacing in the fluorescence imaging sensor.
FIG. 10 is a diagram illustrating an example of a holding member.
FIG. 11 is a diagram illustrating an example of a schematic configuration of the imaging device according to a second embodiment.

FIG. 12 is a graph illustrating an example of a characteristic of a dichroic film 66.

FIG. 13 is a diagram illustrating an example of a schematic configuration of the imaging device according to a third embodiment.

FIG. 14 is a diagram illustrating an example of a characteristic of a dichroic film 67.

FIG. 15 is a diagram illustrating an example of a characteristic of a dichroic film 68.

FIG. 16 is a diagram illustrating an example of a characteristic of a dichroic film 69.

FIG. 17 is a diagram illustrating an example of a schematic configuration of an endoscope system.

FIG. 18 is a block diagram illustrating an example of a functional configuration of a camera and a camera control unit (CCU) illustrated in FIG. 17.

FIG. 19 is a diagram illustrating an example of a schematic configuration of a microscopic surgery system. Description of Embodiments

Embodiments of the present disclosure will be described in detail below with reference to the drawings. Note that in the following embodiments, the same elements will be denoted by the same reference numerals, and the description thereof will not be repeated.

Furthermore, the present disclosure will be described in the order of items shown below.

0. Introduction
1. First Embodiment
2. Second Embodiment
3. Third Embodiment
4. Modifications
5. Application examples
6. Examples of effects

0. Introduction

For example, in a medical endoscope or the like, a wavelength separation prism is used to separate visible light (normal light) and fluorescence (special light) so that corresponding imaging sensors are caused to receive the visible light and the fluorescence. Examples of the visible light include green light, blue light, and red light. Examples of the fluorescence include infrared light (for example, near-infrared light) and the like. Note that hereinafter, the wavelength separation prism is simply referred to as a "prism".

For example, in a three-plate prism configuration in which green light, blue light, red light, and near-infrared light are separated and captured, three monochromatic imaging sensors configured similarly are used. The red light and the near-infrared light are received in a time division manner, and the responsiveness is lowered due to time division. In a two-plate prism configuration in which visible light and fluorescence are separated and captured, the fluorescence is weak (luminance is low), and thus sensitivity is insufficient when the same imaging sensor as the imaging sensor for the visible light is used. Even if it is attempted to observe the fluorescence clearly by increasing the gain or the like, the S/N ratio decreases, and rather, image quality is degraded. In addition, the weak fluorescence causes a flare of the visible light (leak light) that cannot be completely separated by a dichroic film of the prism, decreasing contrast.

At least some of the problems as described above can be addressed by the technology disclosed. For example, the visible light and the fluorescence may be separated according to wavelength by the prism to be simultaneously received by a plurality of imaging sensors having different wavelength band sensitivity (colors or a single color), cell sizes, imaging sizes, and the like. The fluorescence is, for example, near-infrared light weaker than visible light, and such light can be clearly captured. A long-pass filter (trimming filter) may be arranged between the prism and a fluorescence imaging sensor to block visible light that has not been separated according to wavelength by the prism and is unnecessary for capturing fluorescence. The fluorescence imaging sensor may have an imaging size smaller than that of a visible-light imaging sensor. Each of the imaging sensors is arranged at a focus position of an imaging lens system (imaging lens and the like). An imaging device may be used in combination with an LED light source, an excitation laser light source, or the like. The imaging device may be used in combination with a rigid endoscope, a microscope, or the like.

1. First Embodiment

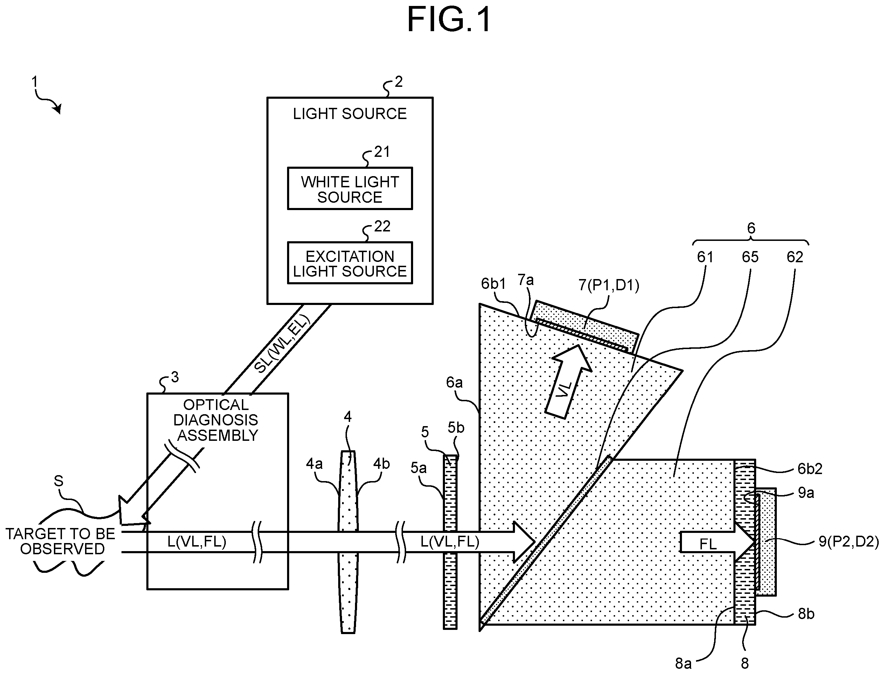

FIG. 1 is a diagram illustrating an example of a schematic configuration of an imaging device according to a first embodiment. The imaging device 1 captures a target S to be observed (subject). A side view of the target S to be observed and elements of the imaging device 1 is schematically illustrated in FIG. 1. The imaging device 1 captures light from the target S to be observed. Note that it may be understood that "capture" includes "shoot", and "image" includes "video". The capture and the image may be appropriately replaced with the shoot and the video, respectively, as long as there is no contradiction.

Observation of the target S to be observed includes visible light observation and fluorescence observation, and thus, capturing the target S to be observed by the imaging device 1 includes capturing visible light and capturing fluorescence. Hereinafter, the fluorescence includes infrared light, unless otherwise described.

The imaging device 1 includes a light source 2, an optical diagnosis assembly 3, an imaging lens 4, an excitation light cut filter 5, a prism 6, a visible-light imaging sensor 7, a long-pass filter 8, and a fluorescence imaging sensor 9. In addition, several rays of light are schematically illustrated by white arrows.

The light source 2 outputs light SL. The light SL includes white light WL and excitation light EL. The excitation light EL is, for example, near-infrared light, and the excitation light EL as the near-infrared light may be laser light having a wavelength of about $780\pm30$ nm. In the example illustrated in FIG. 1, the light source 2 includes a white light source 21 and an excitation light source 22. The white light source 21 outputs the white light WL. The white light source 21 may include a light emitting diode (LED) that emits white light, or may include a light-emitting LED that emits red light, an LED that emits green light, and an LED (RGB-LED) that emits blue light. The excitation light source 22 outputs the excitation light EL.

The optical diagnosis assembly 3 guides the light SL from the light source 2, that is, the white light WL from the white light source 21 and the excitation light EL from the excitation light source 22, to the target S to be observed. The optical diagnosis assembly 3 can constitute part of an imaging system (medical observation system) including, for example, a laparoscope, a rigid endoscope, a flexible endoscope, a camera, a surgical microscope, or the like. The optical diagnosis assembly 3 may include, for example, an optical system for transmitting light, a housing that accommodates the optical system, and the like. The light SL from the light source 2 is applied to the target S to be observed, via the optical diagnosis assembly 3.

The target S to be observed is illuminated by application of the white light WL included in the light SL. In addition, an agent that is excited by application of the excitation light EL included in the light SL is injected into the target S to be observed. Due to these illumination and excitation, light L including visible light VL and fluorescence FL is caused in the target S to be observed. The visible light VL is light reflected from the target S to be observed to which the white light WL is applied. The fluorescence FL is fluorescence emitted from the agent injected into the target S to be observed and excited by the excitation light EL.

The optical diagnosis assembly 3 guides the light L from the target S to be observed, that is, the visible light VL and the fluorescence FL to the prism 6, more specifically, the imaging lens 4 provided on the upstream side (near the target S to be observed) from the prism 6 in this example. The light L from the target S to be observed is input into the imaging lens 4 via the optical diagnosis assembly 3.

The imaging lens 4 is provided between the target S to be observed and the prism 6, more specifically, between the optical diagnosis assembly 3 and the excitation light cut filter 5 in this example. The imaging lens 4 includes, for example, one or more condensing lenses or the like. In the imaging lens 4, an input surface from which the light L from the target S to be observed is input is referred to as an input surface 4a in the drawing. An output surface from which the light L is output is referred to as an output surface 4b in the drawing. The light L from the output surface 4b of the imaging lens 4 is input into the excitation light cut filter 5.

The excitation light cut filter 5 is provided between the target S to be observed and the prism 6, more specifically, between the imaging lens 4 and the prism 6 in this example. When the excitation light EL is included in the light L, the excitation light cut filter 5 reflects or attenuates the excitation light EL and transmits the visible light VL and the fluorescence FL. In the excitation light cut filter 5, a surface from which the light L is input is referred to as an input surface 5a in the drawing. A surface from which the light L is output is referred to as an output surface 5b in the drawing. The light L from the output surface 5b is input into the prism 6. Note that, as long as there is no contradiction, "transmit" may be understood as meaning of "pass", and "transmit" and "pass" may be replaced with each other as appropriate.

The prism 6 separates and outputs the light L from the target S to be observed, more specifically, the visible light VL and the fluorescence FL included in the light L transmitted by the imaging lens 4 and the excitation light cut filter 5 in this example. The prism 6 includes a block 61, a block 62, and a dichroic film 65. In the prism 6, a surface from which the light L is input is referred to as an input surface 6a in the drawing. A surface from which the visible light VL is output is referred to as an output surface 6b1 in the drawing. A surface from which the fluorescence FL is output is referred to as an output surface 6b2 in the drawing. The output surface 6b1 is a part of a surface of the block 61. The output surface 6b2 is a part of a surface of the block 62.

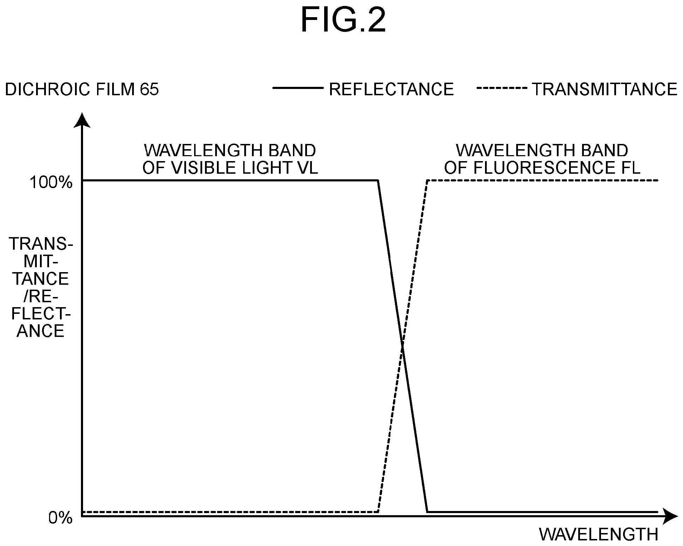

The dichroic film 65 is provided between the block 61 and the block 62 so that the light L from the input surface 6a of the prism 6 is input. In this example, the dichroic film 65 is formed in the block 61. The dichroic film 65 reflects the visible light VL and transmits the fluorescence FL. A description will be given further with reference to FIG. 2.

FIG. 2 is a graph illustrating an example of a characteristic of the dichroic film 65. The horizontal axis of the graph represents wavelength. The vertical axis of the graph represents transmittance and reflectance (%). The reflectance is high in a wavelength band of the visible light VL, and the transmittance is high in a wavelength band of the fluorescence FL.

Returning to FIG. 1, the block 61 of the prism 6 is a prism block (first block) that guides the visible light VL to the visible-light imaging sensor 7. The block 61 outputs the visible light VL reflected by the dichroic film 65, from the output surface 6b1. The block 62 is a prism block (second block) that guides the fluorescence FL to the fluorescence imaging sensor 9. The block 62 outputs the fluorescence FL transmitted by the dichroic film 65, from the output surface 6b2.

The visible light VL output from the output surface 6b1 of the prism 6 is imaged, and is captured by the visible-light imaging sensor 7. The visible-light imaging sensor 7 is directly or indirectly fixed to the prism 6 so as to be located at an imaging position of the visible light VL.

The visible-light imaging sensor 7 captures the visible light VL from the prism 6. The visible-light imaging sensor 7 includes a light receiving surface 7a. The visible-light imaging sensor 7 is provided so that the light receiving surface 7a faces the output surface 6b1 of the prism 6. In this example, the visible-light imaging sensor 7 is provided (bonded or the like) on the output surface 6b1 of the block 61 of the prism 6. The visible-light imaging sensor 7 is, for example, a complementary metal oxide semiconductor (CMOS) image sensor designed to capture the visible light VL.

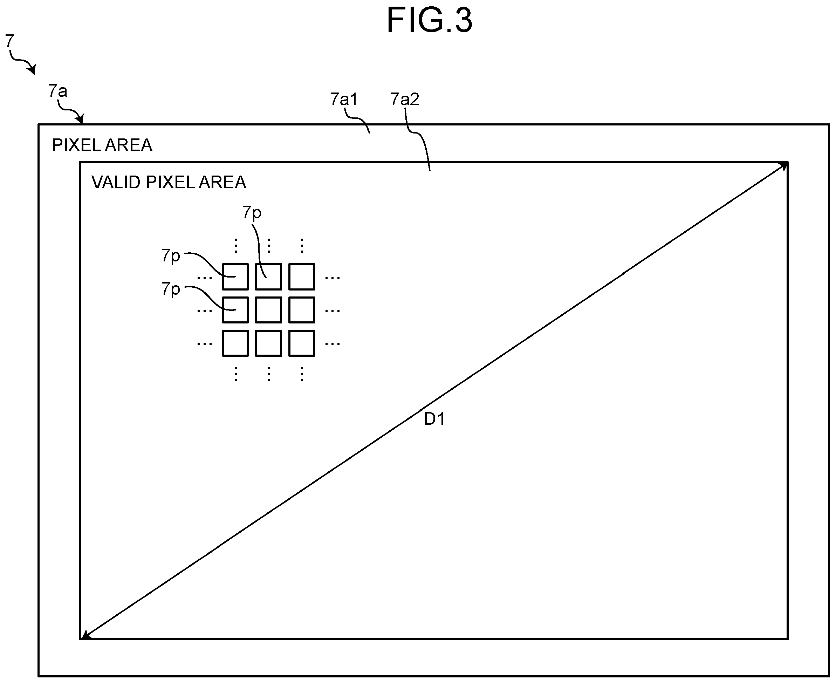

In the first embodiment, a pixel spacing (pixel pitch) in the visible-light imaging sensor 7 is referred to as a pixel spacing P1. A valid pixel area diagonal length of the visible-light imaging sensor 7 is referred to as a valid pixel area diagonal length D1. A description will be further given with reference to FIGS. 3 to 5.

FIG. 3 is a diagram illustrating an example of a schematic configuration of the visible-light imaging sensor. A front view of the visible-light imaging sensor 7 is schematically illustrated. The visible-light imaging sensor 7 includes a plurality of pixels 7p. Each of the pixels 7p is designed to efficiently receive visible light. For example, the plurality of pixels 7p includes a pixel that receives blue light, a pixel that receives red light, and a pixel that receives green light. Each pixel 7p includes a filter that transmits light of corresponding color, a photoelectric conversion element that receives light transmitted by the filter and generates a charge according to an amount of received light, and a transistor or the like (pixel circuit or the like) that controls light reception by the photoelectric conversion element or extracts an electric signal according to the charge. An example of a pixel array includes, but is not limited to, a Bayer array, but various known pixel arrays may be adopted.

The visible-light imaging sensor 7 includes a pixel area 7a1 and a valid pixel area 7a2. The valid pixel area 7a2 is an area inside the pixel area 7a1 and is narrower than the pixel area 7a1. The plurality of pixels 7p is arranged in an array over the entire pixel area 7a1 so as to define the light receiving surface 7a. Of these pixels 7p, pixels 7p arranged in the valid pixel area 7a2 are valid pixels actually used for image capture. The diagonal length of the valid pixel area 7a2 is the valid pixel area diagonal length D1. As the valid pixel area diagonal length D1 increases, the valid pixel area 7a2 also increases. In this sense, the valid pixel area diagonal length D1 may be appropriately replaced with the valid pixel area 7a2, or may be replaced with the meaning of the size of the visible-light imaging sensor 7.

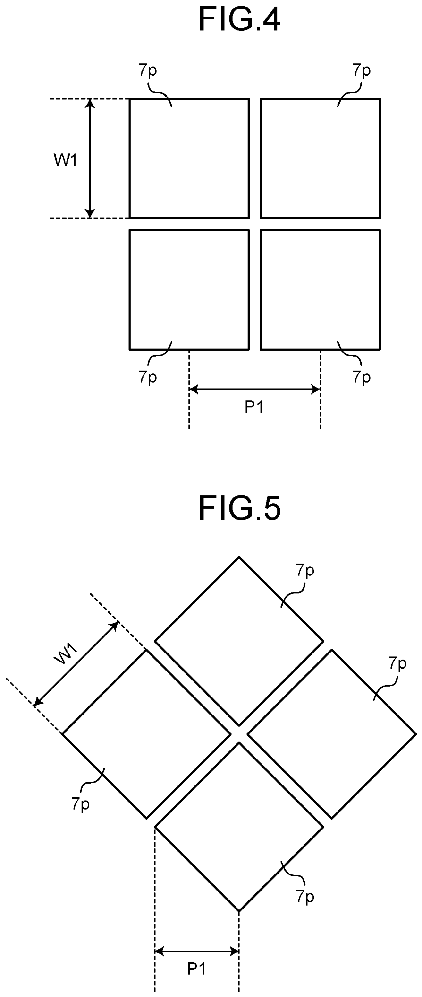

FIGS. 4 and 5 are diagrams each illustrating an example of the pixel spacing in the visible-light imaging sensor. For ease of understanding, only four pixels 7p are illustrated. The pixels 7p may be arranged side by side in a horizontal direction and a vertical direction as illustrated in FIG. 4, or may be arranged side by side in an oblique direction (a direction between the vertical direction and the horizontal direction) as illustrated in FIG. 5. In this example, each of the pixels 7p has a square shape, and a length of one side thereof defines a size of the pixel 7p. The size of the pixel 7p is referred to as a pixel size W1 in the drawing. A distance between the centers of adjacent pixels 7p is the pixel spacing P1. As the pixel spacing P1 increases, the pixel size W1 also increases. In this sense, the pixel spacing P1 may be appropriately replaced with the pixel size W1.

If the valid pixel area diagonal lengths D1 are the same (the visible-light imaging sensors 7 have the same size), the resolution increases as the pixel spacing P1 decreases. The resolution decreases as the pixel spacing P1 increases.

Returning to FIG. 1, the fluorescence FL from the output surface 6b2 of the prism 6 is input into the long-pass filter 8.

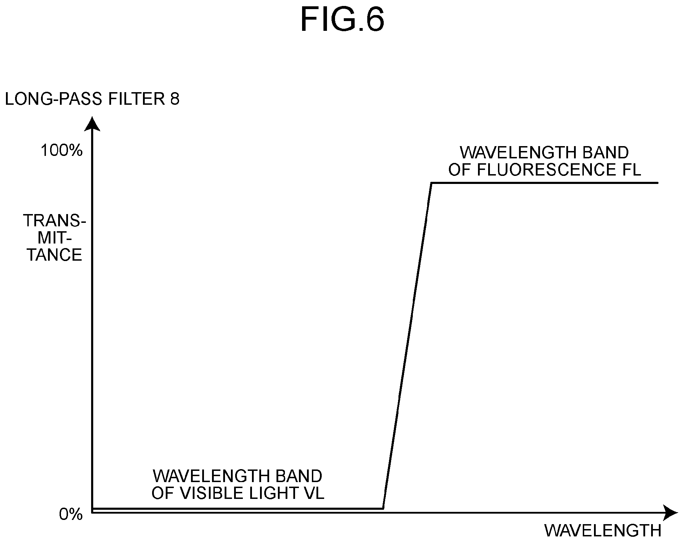

The long-pass filter 8 is provided between the block 62 of the prism 6 and the fluorescence imaging sensor 9. The long-pass filter 8 transmits the fluorescence FL and reflects or attenuates the visible light VL. Unnecessary visible light VL that has not been separated by the prism 6 may cause a flare, and a clear image of fluorescence FL may not be obtained. Providing the long-pass filter 8 suppresses input of such unnecessary visible light VL into the fluorescence imaging sensor 9. A high-quality image having a high S/N ratio can be obtained. In the long-pass filter 8, a surface from which the fluorescence FL is input is referred to as an input surface 8a in the drawing. A surface from which the fluorescence FL is output is referred to as an output surface 8b in the drawing. The long-pass filter 8 will be described further with reference to FIG. 6.

FIG. 6 is a graph illustrating an example of a characteristic of the long-pass filter. The graph shows low transmittance (large amount of reflection or attenuation) in the wavelength band of the visible light VL, and high transmittance (small amount of reflection or attenuation) in the wavelength band of the fluorescence FL. For example, the long-pass filter 8 transmits 90% or more of light having a wavelength of at least 750 nm to 850 nm and attenuates light having a wavelength of 400 nm to 600 nm to 10% or less. More specifically, the long-pass filter 8 may transmit 90% or more of light having a wavelength of at least 820 n to 850 nm and attenuate light having a wavelength of 400 to 600 nm to 10% or less. An optical density of the visible light VL output from the output surface 8b of the long-pass filter 8 to the visible light VL input into the input surface 6a of the prism 6 may be OD3 or more.

Returning to FIG. 1, the input surface 8a of the long-pass filter 8 may be a surface perpendicular to an optical axis.

The long-pass filter 8 is provided at the prism 6 so that the input surface 8a faces the output surface 6b2 of the block 62 of the prism 6. In the example illustrated in FIG. 1, the long-pass filter 8 is provided (for example, bonded) on the output surface 6b2 of the block 62 of the prism 6.

The fluorescence FL from the output surface 6b2 of the prism 6, more specifically, the fluorescence FL from the output surface 8b of the long-pass filter 8 in this example is imaged, and is captured by the fluorescence imaging sensor 9. The fluorescence imaging sensor 9 is directly or indirectly fixed to the prism 6 so as to be located at an imaging position of the fluorescence FL.

The fluorescence imaging sensor 9 captures the fluorescence FL from the prism 6. The fluorescence imaging sensor 9 includes a light receiving surface 9a. The fluorescence imaging sensor 9 is provided so that the light receiving surface 9a faces the output surface 6b2 of the prism 6. In this example, the fluorescence imaging sensor 9 is provided (for example, bonded) on the output surface 8b of the long-pass filter 8 so as to face the output surface 8b of the long-pass filter 8. The fluorescence imaging sensor 9 is, for example, a complementary metal-oxide-semiconductor (CMOS) imaging sensor designed to capture the fluorescence FL.

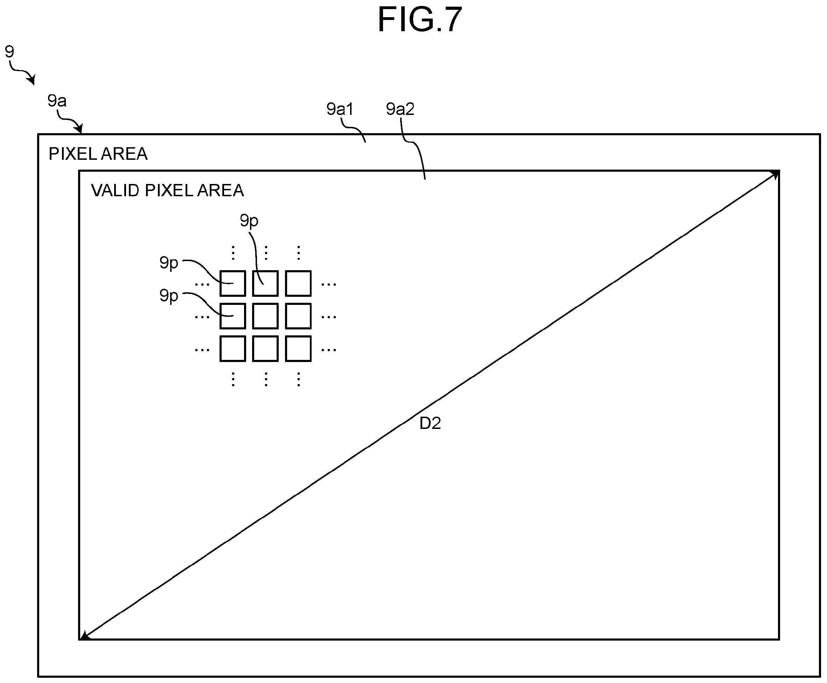

A pixel spacing in the fluorescence imaging sensor 9 is referred to as a pixel spacing P2. A valid pixel area diagonal length of the fluorescence imaging sensor 9 is referred to as a valid pixel area diagonal length D2. A description will be further given with reference to FIGS. 7 to 9.

FIG. 7 is a diagram illustrating an example of a schematic configuration of the fluorescence imaging sensor. A front view of the fluorescence imaging sensor 9 is schematically illustrated. The fluorescence imaging sensor 9 includes a plurality of pixels 9p. Each of the pixels 9p is designed to efficiently receive the fluorescence FL. Each pixel 9p includes a filter that transmits the fluorescence FL, a photoelectric conversion element that receives light transmitted by the filter and generates a charge according to an amount of received light, and a transistor or the like (pixel circuit or the like) that controls light reception by the photoelectric conversion element or extracts an electric signal according to the charge. Various known pixel arrays may be adopted for a pixel array.

The fluorescence imaging sensor 9 includes a pixel area 9a1 and a valid pixel area 9a2. The plurality of pixels 9p is arranged in an array over the entire pixel area 9a1 so as to define the light receiving surface 9a. Of these pixels 9p, pixels 9p arranged in the valid pixel area 9a2 are valid pixels actually used for image capture. A diagonal length of the valid pixel area 9a2 is the valid pixel area diagonal length D2. If the valid pixel area diagonal lengths D2 are the same (the fluorescence imaging sensors 9 have the same size), the resolution increases as the pixel spacing P2 decreases. As the pixel spacing P2 increases, the resolution decreases.

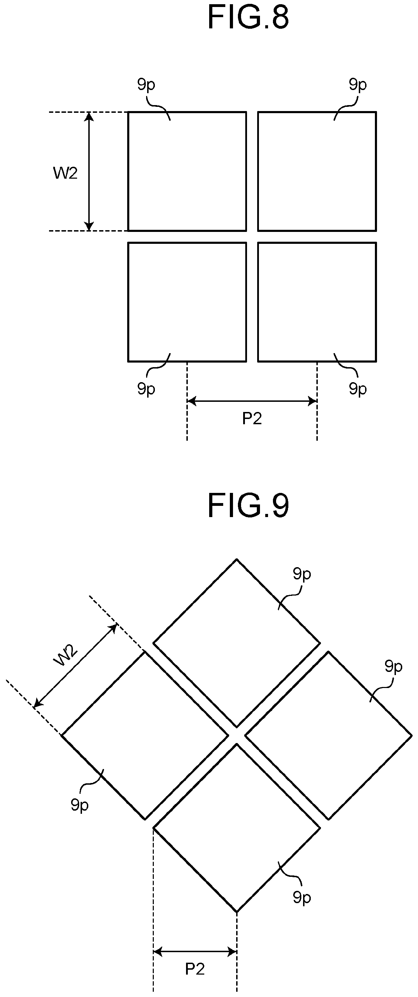

FIGS. 8 and 9 are diagrams each illustrating an example of the pixel spacing in the fluorescence imaging sensor. The pixels 9p may be arranged side by side in a horizontal direction and a vertical direction as illustrated in FIG. 8, or may be arranged side by side in an oblique direction as illustrated in FIG. 9. The size of the pixel 9p is referred to as a pixel size W2 in the drawing. A distance between the centers of adjacent pixels 9p is the pixel spacing P2. As the pixel size W2 increases, the pixel spacing P2 also increases.

Returning to FIG. 1, in the imaging device 1 described above, the visible light VL and the fluorescence FL from the target S to be observed are separated by the prism 6 and captured by the visible-light imaging sensor 7 and the fluorescence imaging sensor 9, respectively, and the visible light observation and the fluorescence observation of the target S to be observed are performed. Here, there is a possibility that the sensitivity of the fluorescence imaging sensor 9 is insufficient and clear fluorescence observation cannot be made, because of the fluorescence FL weaker than the visible light VL, or the like. The imaging device 1 according to the first embodiment is designed to capture the visible light VL with high resolution and to capture the fluorescence FL clearly. Several design conditions will be described. Note that of the design conditions described below, non-exclusive design conditions may be appropriately combined.

<Design condition 1-1>

In one embodiment, a ratio (P2/P1) of the pixel spacing P2 in the fluorescence imaging sensor 9 to the pixel spacing P1 in the visible-light imaging sensor 7 may be 1 or more and less than 5. In other words, the pixel spacing P1 in the visible-light imaging sensor 7 and the pixel spacing P2 in the fluorescence imaging sensor 9 may be designed to satisfy the following conditional expression (1).

$$1 \leqq \frac{P2}{P1} < 5 \quad \cdots (1)$$

When the ratio (P2/P1) falls below 1, the pixel size W2 of the fluorescence imaging sensor 9 decreases, the weak fluorescence FL captured into each pixel 9p decreases in the amount of light, and noise in the captured image increases. When the ratio (P2/P1) is 5 or more, the pixel size W2 of the fluorescence imaging sensor 9 increases, and the imaging device 1 increases in size. When the number of pixels 9p is reduced to prevent the increase in size of the imaging device 1, the resolution decreases.

When the ratio (P2/P1) is larger than 1, the pixel size W2 of the fluorescence imaging sensor 9 increases and the amount of light input increases, and it is possible to receive the weak fluorescence FL with high sensitivity to clearly capture the fluorescence FL. When the ratio (P2/P1) is 1, for example, using a color imaging sensor as the visible-light imaging sensor 7 and a monochrome imaging sensor as the fluorescence imaging sensor 9 makes it possible to receive the fluorescence FL with high sensitivity and capture the fluorescence FL clearly, while maintaining the resolution equally.

Note that a lower limit value of the ratio (P2/P1) may be larger than the value represented in the conditional expression (1) described above. Examples of such lower limit values are 1.1, 1.2, 1.3, 1.4, 1.5, 1.6, 1.7, 1.8, 1.9, 2.0, 2.1, 2.2, 2.3, 2.4, 2.5, and the like. Furthermore, an upper limit value thereof may be smaller than the value represented in the conditional expression (1) described above. Examples of such upper limit values are 4.8, 4.6, 4.4, 4.2, 4.0, 3.8, 3.6, 3.4, 3.2, 3.0, 2.8, 2.6, 2.4, 2.2, 2.0, 1.8, 1.6, and the like.

<Design condition 1-2>

In one embodiment, a ratio (D2/D1) of the valid pixel area diagonal length D2 of the fluorescence imaging sensor 9 to the valid pixel area diagonal length D1 of the visible-light imaging sensor 7 may be larger than 0.7 and less than 1.4. In other words, the valid pixel area diagonal length D1 of the visible-light imaging sensor 7 and the valid pixel area diagonal length D2 of the fluorescence imaging sensor 9 may be designed to satisfy the following conditional expression (2).

$$0.7 < \frac{D2}{D1} < 1.4 \quad \cdots (2)$$

When the ratio (D2/D1) is 0.7 or less, an angle of view of the fluorescence imaging sensor 9 is significantly reduced compared with an angle of view of the visible-light imaging sensor 7, and a field of view in which the fluorescence FL can be observed is limited. When the ratio (D2/D1) is 1.4 or more, the angle of view of the fluorescence imaging sensor 9 is significantly increased compared with the angle of view of the visible-light imaging sensor 7, and pixels 9p that are not used are generated in the valid pixel area 9a2 of the fluorescence imaging sensor 9.

When the ratio (D2/D1) is smaller than 1, the fluorescence imaging sensor 9 is relatively small with respect to the prism 6, and there is room for an optical path. The unnecessary light generated by the reflection from members constituting the prism 6 and input into the fluorescence imaging sensor 9 can be reduced, and the weak fluorescence FL can be clearly captured. For example, an unnecessary light component such as the flare appearing with increase of the sensitivity of the fluorescence imaging sensor 9 is reduced, and a clear fluorescent image can be obtained. When the ratio (D2/D1) is larger than 1, the pixel size W2 can be increased while keeping a relatively large number of pixels 9p, and the weak fluorescence FL can be captured with high sensitivity and high resolution. When the ratio (D2/D1) is 1, increasing the pixel spacing P2 in the fluorescence imaging sensor 9 makes it possible to increase the pixel size W2 for high sensitivity, and further, make accompanying components common for facilitation of assemblability of the imaging device 1.

Note that a lower limit value of the ratio (D2/D1) may be larger than the value represented in the conditional expression (2) described above. Examples of such lower limit values are 0.8, 0.9, 1.0, and the like. Note that an upper limit value thereof may be smaller than the value represented in the conditional expression (2). Examples of such upper limit values are 1.3, 1.2, 1.1, and the like.

<Design condition 1-3>

Optical path lengths of the visible light VL and the fluorescence FL, from the input into the prism 6 to the imaging, can be different from each other, depending on the characteristics of the optical diagnosis assembly 3 and the imaging lens 4, the wavelengths of the respective light, and the like. In that case, the visible-light imaging sensor 7 may be located at the imaging position of the visible light VL and the fluorescence imaging sensor 9 may be located at the imaging position of the fluorescence FL so as to suppress the influence of a difference between the optical path lengths (optical path length difference), that is, so as to absorb an amount of deviation in the optical path length difference. This configuration only requires focusing of the visible-light imaging sensor 7 to focus the fluorescence imaging sensor 9, and facilitates capturing the visible light VL with high resolution and capturing the fluorescence FL clearly. Several examples of specific positioning methods will be described.

<Positioning method 1-1>

In one embodiment, the long-pass filter 8 may have an optical axial length designed according to the optical path length difference described above so as to position the fluorescence imaging sensor 9 at the imaging position of the fluorescence FL. When the input surface 8a of the long-pass filter 8 is perpendicular to the optical axis, the optical axial length of the long-pass filter 8 corresponds to a thickness of the long-pass filter 8. Adjusting the thickness of the long-pass filter 8 makes it possible to bring the fluorescence imaging sensor 9 closer to or away from the prism 6.

<Positioning method 1-2>

In one embodiment, the prism 6 may have optical axial lengths designed according to the optical path length difference described above so as to position the visible-light imaging sensor 7 at the imaging position of the visible light VL and position the fluorescence imaging sensor 9 at the imaging position of the fluorescence FL. For example, adjusting an optical axial length of the block 61 makes it possible to bring the visible-light imaging sensor 7 closer to or away from the prism 6. Adjusting an optical axial length of the block 62 makes it possible to bring the fluorescence imaging sensor 9 closer to or away from the prism 6.

<Positioning method 1-3>

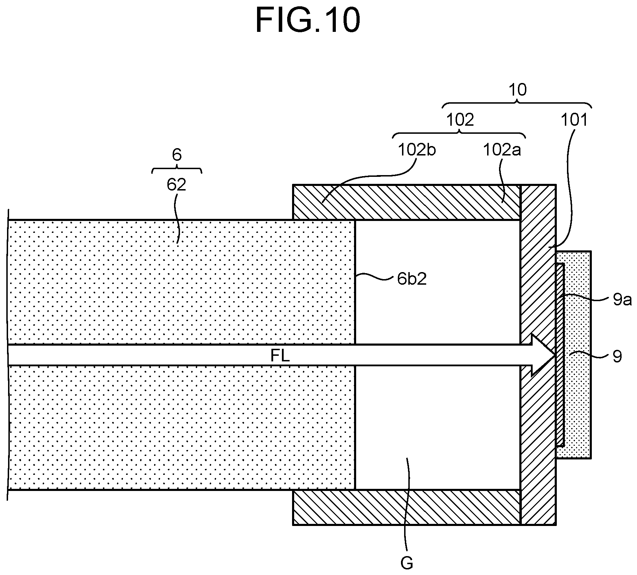

In one embodiment, the imaging device 1 may include a gap positioned between the prism 6 and at least one of the visible-light imaging sensor 7 and the fluorescence imaging sensor 9. The gap may have optical axial lengths designed according to the optical path length difference described above so as to position the visible-light imaging sensor 7 at the imaging position of the visible light VL and position the fluorescence imaging sensor 9 at the imaging position of the fluorescence FL. A holding member for providing the gap configured as described above may be used. A description will be given with reference to FIG. 10.

FIG. 10 is a diagram illustrating an example of the holding member. In this example, the holding member 10 provides a gap G positioned between the block 62 of the prism 6 and the fluorescence imaging sensor 9. Note that the long-pass filter 8 (FIG. 1) is not illustrated. The gap G can also be said as an air gap between the block 62 and the fluorescence imaging sensor 9. The holding member 10 holds the fluorescence imaging sensor 9 so as to provide the gap G. Specifically, in this example, the holding member 10 includes a base plate 101 and a holding rod 102.

The base plate 101 is bonded on the light receiving surface 9a of the fluorescence imaging sensor 9 to support the fluorescence imaging sensor 9. An example of a material of the base plate 101 is glass or the like, and the fluorescence FL transmitted by the base plate 101 is input into the light receiving surface 9a of the fluorescence imaging sensor 9. The holding rod 102 includes one end portion 102a that is connected to an edge portion of the base plate 101 and the other end portion 102b that holds a side surface of the block 62 of the prism 6. The holding rod 102 has a length that gives the optical axial length of the gap G. Adjusting the length of the holding rod 102 makes it possible to bring the fluorescence imaging sensor 9 closer to or away from the prism 6.

Note that the imaging device 1 may include the holding member 10 that holds the visible-light imaging sensor 7, instead of or in addition to the fluorescence imaging sensor 9 that holds the holding member 10.

2. Second Embodiment

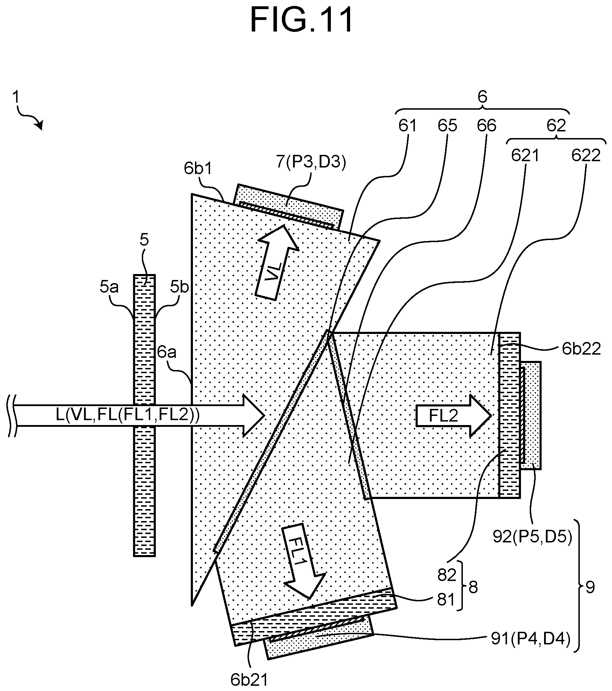

FIG. 11 is a diagram illustrating an example of a schematic configuration of the imaging device according to a second embodiment. FIG. 11 does not illustrate the target S to be observed, the light source 2, the optical diagnosis assembly 3, and the imaging lens 4 of FIG. 1 which have been described above. Differences from the first embodiment will be mainly described. The prism 6 further includes a dichroic film 66. The block 62 of the prism 6 includes a block 621 and a block 622. The long-pass filter 8 includes a long-pass filter 81 and a long-pass filter 82. The fluorescence imaging sensor 9 includes a fluorescence imaging sensor 91 and a fluorescence imaging sensor 92.

The fluorescence FL included in the light L input into the prism 6 includes fluorescence FL1 and fluorescence FL2. The fluorescence FL1 and the fluorescence FL2 are first fluorescence and second fluorescence that belong to different wavelength bands. For example, the fluorescence FL1 is near-infrared light, and the fluorescence FL2 is light on the longer wavelength side relative to the near-infrared light. Note that the excitation light source 22 (FIG. 1) of the light source 2 may output two types of excitation light that excite the target S to be observed so that the agent injected into the target S to be observed emits the fluorescence FL1 and the fluorescence FL2. For example, two types of agents, that is, an agent that emits fluorescence FL1 and an agent that emits fluorescence FL2, are injected into the target S to be observed.

The prism 6 separates and outputs the visible light VL, the fluorescence FL1, and the fluorescence FL2. The block 621 is positioned between the block 61 and the block 622. A surface of the prism 6 from which the fluorescence FL1 is output is referred to as an output surface 6b21 in the drawing. A surface from which the fluorescence FL2 is output is referred to as an output surface 6b22 in the drawing. The output surface 6b21 is a part of a surface of the block 621. The output surface 6b22 is a part of a surface of the block 622.

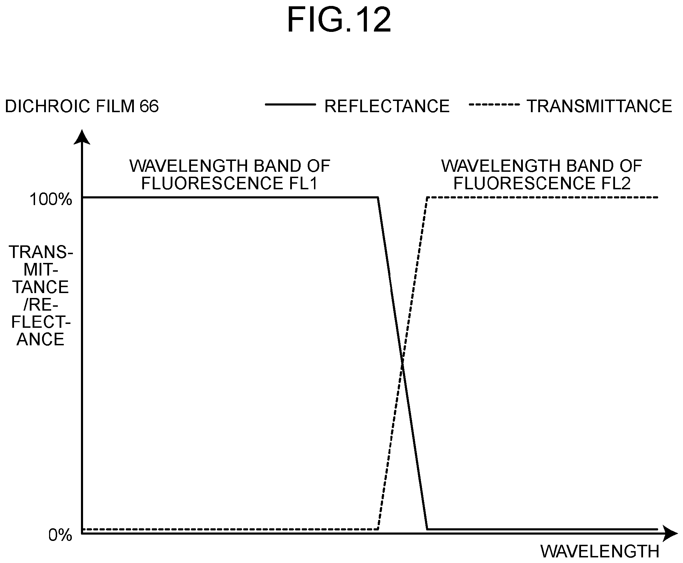

The dichroic film 66 is provided between the block 621 and the block 622 so that the fluorescence FL1 and the fluorescence FL2 transmitted by the dichroic film 65 are input. In this example, the dichroic film 66 is formed in the block 621. The dichroic film 66 reflects the fluorescence FL1 and transmits the fluorescence FL2. A description will be given further with reference to FIG. 12.

FIG. 12 is a graph illustrating an example of a characteristic of the dichroic film 66. Reflectance is high in a wavelength band including at least the fluorescence FL1, and transmittance is high in a wavelength band including at least the fluorescence FL2.

Returning to FIG. 11, the block 621 outputs the fluorescence FL1 reflected by the dichroic film 66, from the output surface 6b21. The block 622 outputs the fluorescence FL2 transmitted by the dichroic film 66, from the output surface 6b22.

The long-pass filter 81 is provided between the block 621 and the fluorescence imaging sensor 91. The long-pass filter 81 transmits the fluorescence FL1 and reflects or attenuates light having a wavelength shorter than the fluorescence FL1. The long-pass filter 82 is provided between the block 622 and the fluorescence imaging sensor 92. The long-pass filter 82 transmits the fluorescence FL2 and reflects or attenuates light having a wavelength shorter than the fluorescence FL2.

The fluorescence FL1 from the long-pass filter 81 is imaged, and is captured by the fluorescence imaging sensor 91. The fluorescence imaging sensor 91 may be a first fluorescence imaging sensor designed to capture the fluorescence FL1 with high sensitivity. The fluorescence imaging sensor 91 is directly or indirectly fixed to the prism 6 so as to be located at an imaging position of the fluorescence FL1.

The fluorescence FL2 from the long-pass filter 82 is imaged, and is captured by the fluorescence imaging sensor 92. The fluorescence imaging sensor 92 may be a second fluorescence imaging sensor designed to capture the fluorescence FL2 with high sensitivity. The fluorescence imaging sensor 92 is directly or indirectly fixed to the prism 6 so as to be located at an imaging position of the fluorescence FL2.

In the second embodiment, a pixel spacing and a valid pixel area diagonal length of the visible-light imaging sensor 7 are referred to as a pixel spacing P3 and a valid pixel area diagonal length D3, respectively. A pixel spacing and a valid pixel area diagonal length of the fluorescence imaging sensor 91 are referred to as a pixel spacing P4 and a valid pixel area diagonal length D4, respectively. A pixel spacing and a valid pixel area diagonal length of the fluorescence imaging sensor 92 are referred to as a pixel spacing P5 and a valid pixel area diagonal length D5, respectively.

The imaging device 1 according to the second embodiment is designed to capture the visible light VL with high resolution and to capture the fluorescence FL1 and the fluorescence FL2 clearly. Several design conditions will be described. Note that of the design conditions described below, non-exclusive design conditions may be appropriately combined.

<Design condition 2-1>

In one embodiment, both if a ratio (P4/P3) of the pixel spacing P4 in the fluorescence imaging sensor 91 to the pixel spacing P3 in the visible-light imaging sensor 7 and a ratio (P5/P3) of the pixel spacing P5 in the fluorescence imaging sensor 92 to the pixel spacing P3 in the visible-light imaging sensor 7 may be 1 or more and less than 5. In other words, the pixel spacing P3 in the visible-light imaging sensor 7, the pixel spacing P4 in the fluorescence imaging sensor 91, and the pixel spacing P5 in the fluorescence imaging sensor 92 may be designed to satisfy the following conditional expression (3). The technical significance is similar to that of the conditional expression (1) described in the above first embodiment, and the description thereof will not be repeated. The same applies to the point that the lower limit value may be larger than the value represented by the conditional expression and the point that the upper limit value may be smaller than the value represented by the conditional expression.

$$1 \leqq \frac{P4}{P3} < 5 \ and \ 1 \leqq \frac{P5}{P3} < 5 \qquad \cdots (3)$$

<Design condition 2-2>

In one embodiment, both of a ratio (D4/D3) of the valid pixel area diagonal length D4 of the fluorescence imaging sensor 91 to the valid pixel area diagonal length D3 of the visible-light imaging sensor 7 and a ratio (D5/D3) of the valid pixel area diagonal length D5 of the fluorescence imaging sensor 92 to the valid pixel area diagonal length D3 of the visible-light imaging sensor 7 may be larger than 0.7 and smaller than 1.4. In other words, the valid pixel area diagonal length D3 of the visible-light imaging sensor 7, the valid pixel area diagonal length D4 of the fluorescence imaging sensor 91, and the valid pixel area diagonal length D5 of the fluorescence imaging sensor 92 may be designed to satisfy the following conditional expression (4). The technical significance is similar to that of the conditional expression (2) described in the above first embodiment, and the description thereof will not be repeated. The same applies to the point that the lower limit value may be larger than the value represented by the conditional expression and the point that the upper limit value may be smaller than the value represented by the conditional expression.

$$0.7 < \frac{D4}{D3} < 1.4 \ \ and \ \ 0.7 < \frac{D5}{D3} < 1.4 \qquad \cdots (4)$$

<Design condition 2-3>

Optical path lengths of the visible light VL, the fluorescence FL1, and the fluorescence FL2, from the input into the prism 6 to the imaging, can be different from each other, depending on the characteristics of the optical diagnosis assembly 3 and the imaging lens 4, the wavelengths of the respective light, and the like. In that case, the visible-light imaging sensor 7 may be located at the imaging position of the visible light VL, the fluorescence imaging sensor 91 may be located at the imaging position of the fluorescence FL1, and the fluorescence imaging sensor 92 may be located at the imaging position of the fluorescence FL2 so as to suppress the influence of the optical path length difference. This configuration only requires focusing of the visible-light imaging sensor 7 to focus the fluorescence imaging sensor 91 and the fluorescence imaging sensor 92, and facilitates capturing the visible light VL with high resolution and capturing the fluorescence FL1 and the fluorescence FL2 clearly. , Several examples of specific positioning methods will be described.

<Positioning method 2-1>

In one embodiment, the long-pass filter 81 may have an optical axial length designed according to, for example, an optical path length difference between the visible light VL and the fluorescence FL1 so as to position the fluorescence imaging sensor 91 at the imaging position of the fluorescence FL1. Similarly, the long-pass filter 82 may have an optical axial length designed according to, for example, an optical path length difference between the visible light VL and the fluorescence FL2 so as to position the fluorescence imaging sensor 92 at the imaging position of the fluorescence FL2.

<Positioning method 2-2>

In one embodiment, the prism 6 may have optical axial lengths designed according to the optical path length difference described above so as to position the visible-light imaging sensor 7 at the imaging position of the visible light VL, position the fluorescence imaging sensor 91 at the imaging position of the fluorescence FL1, and position the fluorescence imaging sensor 92 at the coupling position of the fluorescence FL2. For example, adjusting an optical axial length of the block 61 makes it possible to bring the visible-light imaging sensor 7 closer to or away from the prism 6. Adjusting an optical axial length of the block 621 makes it possible to bring the fluorescence imaging sensor 91 closer to or away from the prism 6. Adjusting an optical axial length of the block 622 makes it possible to bring the fluorescence imaging sensor 92 closer to or away from the prism 6.

<Positioning method 2-3>

In one embodiment, the imaging device 1 may include a gap positioned between the prism 6 and at least one of the visible-light imaging sensor 7, the fluorescence imaging sensor 91, and the fluorescence imaging sensor 92. The gap may have optical axial lengths designed according to the optical path length difference described above so as to position the visible-light imaging sensor 7 at the imaging position of the visible light VL, position the fluorescence imaging sensor 91 at the imaging position of the fluorescence FL1, and position the fluorescence imaging sensor 92 at the imaging position of the fluorescence FL2. A holding member for providing the gap configured as described above may be used. The gap and the holding member may have similar configurations to the gap and the holding member described in the first embodiment with reference to FIG. 10.

3. Third Embodiment

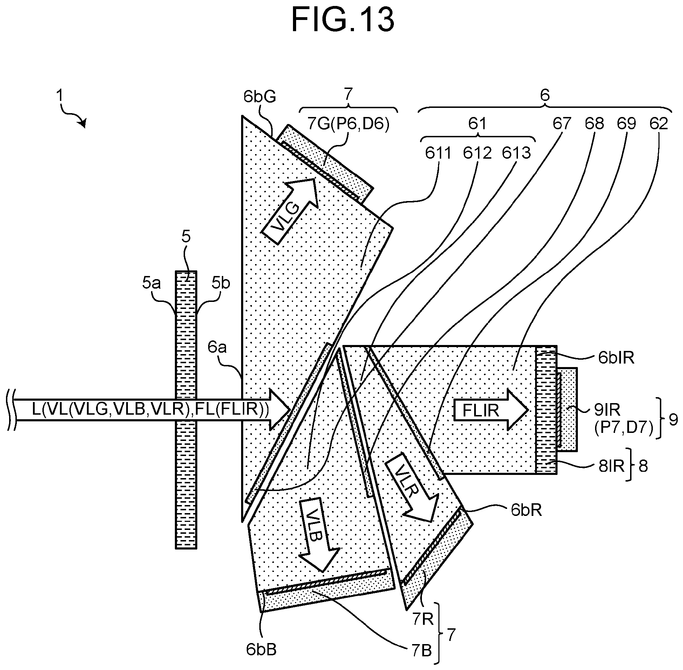

FIG. 13 is a diagram illustrating an example of a schematic configuration of the imaging device according to a third embodiment. FIG. 13 does not illustrate the target S to be observed, the light source 2, the optical diagnosis assembly 3, and the imaging lens 4 of FIG. 1 which have been described above. Differences from the first embodiment will be mainly described. The prism 6 does not include the dichroic film 65 (FIG. 1), but includes a dichroic film 67, a dichroic film 68, and a dichroic film 69. The block 61 of the prism 6 includes a block 611, a block 612, and a block 613. The blocks 611 and 612 may be arranged so that air is interposed therebetween. Similarly, the blocks 612 and 613 may be arranged so that air is interposed therebetween. The visible-light imaging sensor 7 includes a green light imaging sensor 7G, a blue light imaging sensor 7B, and a red light imaging sensor 7R. The long-pass filter 8 includes a long-pass filter 8IR. The fluorescence imaging sensor 9 includes an infrared imaging sensor 9IR.

The visible light VL included in the light L input into the prism 6 includes green light VLG, blue light VLB, and red light VLR. The fluorescence FL includes infrared light FLIR.

The prism 6 separates and outputs the green light VLG, the blue light VLB, the red light VLR, and the infrared light FLIR. The block 612, the block 613, and the block 62 are positioned in series relative to the block 61. The block 613 is positioned between the block 612 and the block 62. A surface of the prism 6 from which the green light VLG is output is referred to as an output surface 6bG in the drawing. A surface from which the blue light VLB is output is referred to as an output surface 6bB in the drawing. A surface from which the red light VLR is output is referred to as an output surface 6bR in the drawing. A surface from which the infrared light FLIR is output is referred to as an output surface 6bIR in the drawing. The output surface 6bG is a part of a surface of the block 611. The output surface 6bB is a part of a surface of the block 612. The output surface 6bR is a part of a surface of the block 613. The output surface 6bIR is part of a surface of the block 62.

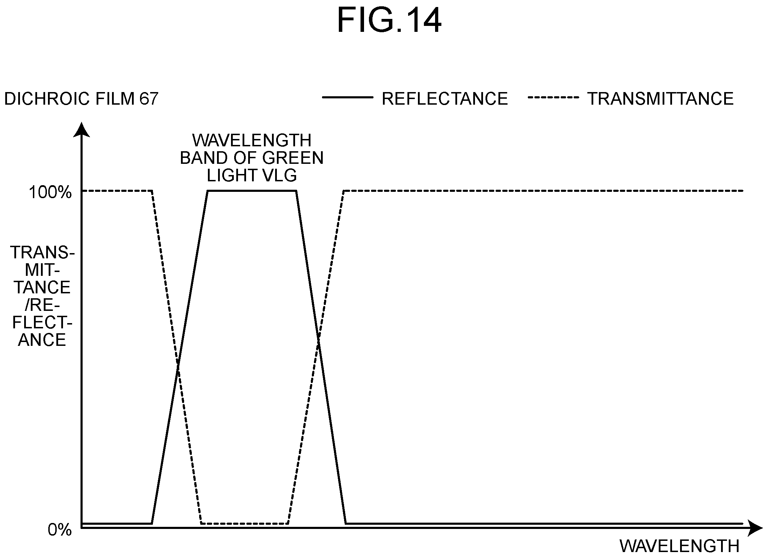

The dichroic film 67 is provided between the block 611 and the block 612 so that the light L from the input surface 6a of the prism 6 is input. In this example, the dichroic film 67 is formed in the block 611. The dichroic film 67 reflects the green light VLG and transmits other light. A description will be given further with reference to FIG. 14.

FIG. 14 is a diagram illustrating an example of a characteristic of the dichroic film 67. Reflectance is high in a wavelength band of the green light VLG, and transmittance is high in the other wavelength bands.

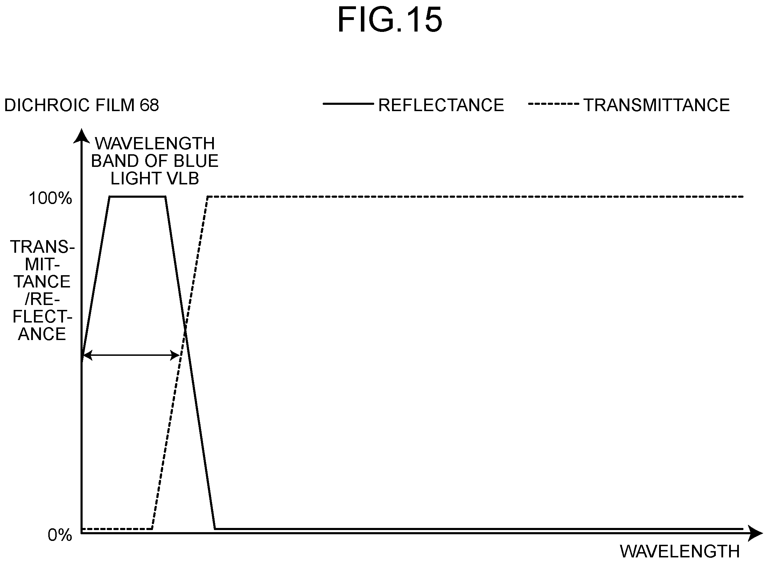

Returning to FIG. 13, the dichroic film 68 is provided between the block 612 and the block 613. In this example, the dichroic film 68 is formed in the block 612. The dichroic film 68 reflects the blue light VLB and transmits other light. A description will be given further with reference to FIG. 15.

FIG. 15 is a diagram illustrating an example of a characteristic of the dichroic film 68. Reflectance is high in a wavelength band of the blue light VLB, and transmittance is high in the other wavelength bands.

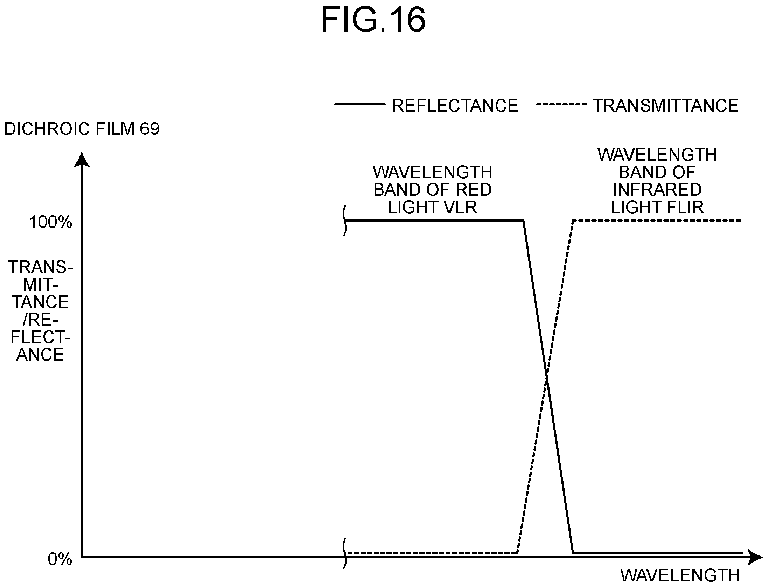

Returning to FIG. 13, the dichroic film 69 is provided between the block 613 and the block 62. In this example, the dichroic film 69 is formed in the block 613. The dichroic film 69 reflects the red light VLR and transmits the infrared light FLIR. A description will be given further with reference to FIG. 16.

FIG. 16 is a diagram illustrating an example of a characteristic of the dichroic film 69. Reflectance is high in a wavelength band of the red light VLR, and transmittance is high in a wavelength band of the infrared light FLIR.

Returning to FIG. 13, the block 611 outputs the green light VLG reflected by the dichroic film 67, from the output surface 6bG. The block 612 outputs the blue light VLB transmitted by the dichroic film 67 and reflected by the dichroic film 68, from the output surface 6bB. The block 613 outputs the red light VLR transmitted by the dichroic film 68 and reflected by the dichroic film 69, from the output surface 6bR. The block 62 outputs light (light including the infrared light FLIR) transmitted by the dichroic film 69, from the output surface 6bIR.

The green light VLG output from the output surface 6bG of the prism 6 is imaged, and is captured by the green light imaging sensor 7G. The green light imaging sensor 7G may be a visible-light imaging sensor designed to capture the green light VLG with high sensitivity and high resolution. For example, each of pixels included in the green light imaging sensor 7G is configured to receive green light. The green light imaging sensor 7G is directly or indirectly fixed to the prism 6 so as to be located at an imaging position of the green light VLG.

The blue light VLB output from the output surface 6bB of the prism 6 is imaged, and is captured by the blue light imaging sensor 7B. The blue light imaging sensor 7B may be a visible-light imaging sensor designed to capture the blue light VLB with high sensitivity and high resolution. For example, each of pixels included in the blue light imaging sensor 7B is configured to receive blue light. The blue light imaging sensor 7B is directly or indirectly fixed to the prism 6 so as to be located at an imaging position of the blue light VLB.

The red light VLR output from the output surface 6bR of the prism 6 is imaged, and is captured by the red light imaging sensor 7R. The red light imaging sensor 7R may be a visible-light imaging sensor designed to capture the red light VLR with high sensitivity and high resolution. For example, each of pixels included in the red light imaging sensor 7R is configured to receive red light. The red light imaging sensor 7R is directly or indirectly fixed to the prism 6 so as to be located at an imaging position of the red light VLR.

The long-pass filter 8IR is provided between the block 62 and the infrared imaging sensor 9IR. The long-pass filter 8IR transmits the infrared light FLIR and reflects or attenuates light having a wavelength shorter than the infrared light FLIR.

The infrared light FLIR from the output surface 6bIR of the prism 6, more specifically, the infrared light FLIR from the long-pass filter 8IR in this example is imaged, and is captured by the infrared imaging sensor 9IR. The infrared imaging sensor 9IR may be an infrared imaging sensor designed to capture the infrared light FLIR from the long-pass filter 8IR with high sensitivity. The infrared imaging sensor 9IR is directly or indirectly fixed to the prism 6 so as to be located at an imaging position of the infrared light FLIR.

In the third embodiment, a pixel spacing and a valid pixel area diagonal length of the green light imaging sensor 7G are referred to as a pixel spacing P6 and a valid pixel area diagonal length D6, respectively. A pixel spacing and a valid pixel area diagonal length of the infrared imaging sensor 9IR are referred to as a pixel spacing P7 and a valid pixel area diagonal length D7, respectively. Note that pixel spacings and valid pixel area diagonal lengths of the blue light imaging sensor 7B and the red light imaging sensor 7R may be the same as, for example, the pixel spacing P6 and the valid pixel area diagonal length D6 of the green light imaging sensor 7G.

The imaging device 1 according to the third embodiment is designed to capture the green light VLG, the blue light VLB, and the red light VLR with high resolution and capture the infrared light FLIR clearly. Several design conditions will be described. Note that of the design conditions described below, non-exclusive design conditions may be appropriately combined.

<Design condition 3-1>

In one embodiment, a ratio (P7/P6) of the pixel spacing P7 in the infrared imaging sensor 9IR to the pixel spacing P6 in the green light imaging sensor 7G may be 1 or more and less than 5. In other words, the pixel spacing P6 in the green light imaging sensor 7G and the pixel spacing P7 in the infrared imaging sensor 9IR may be designed to satisfy the following conditional expression (5). The technical significance is similar to that of the conditional expression (1) described in the above first embodiment, and the description thereof will not be repeated. The same applies to the point that the lower limit value may be larger than the value represented by the conditional expression and the point that the upper limit value may be smaller than the value represented by the conditional expression.

$$1 \leqq \frac{P7}{P6} < 5 \quad \cdots (5)$$

<Design condition 3-2>

In one embodiment, a ratio (D7/D6) of the valid pixel area diagonal length D7 of the infrared imaging sensor 9IR to the valid pixel area diagonal length D6 of the green light imaging sensor 7G may be larger than 0.7 and less than 1.4. In other words, the valid pixel area diagonal length D6 of the green light imaging sensor 7G and the valid pixel area diagonal length D7 of the infrared imaging sensor 9IR may be designed to satisfy the following conditional expression (6). The technical significance is similar to that of the conditional expression (2) described in the above first embodiment, and the description thereof will not be repeated. The same applies to the point that the lower limit value may be larger than the value represented by the conditional expression and the point that the upper limit value may be smaller than the value represented by the conditional expression.

$$0.7 < \frac{D7}{D6} < 1.4 \quad \cdots (6)$$

<Design condition 3-3>

Optical path lengths of the green light VLG and the infrared light FLIR, from the input into the prism 6 to the imaging, can be different from each other, depending on the characteristics of the imaging lens 4, the wavelengths of the respective light, and the like. The green light imaging sensor 7G may be located at the imaging position of the green light VLG, and the infrared imaging sensor 9IR may be located at the imaging position of the infrared light FLIR so as to suppress the influence of the optical path length difference. This configuration only requires focusing of the green light imaging sensor 7G to focus the infrared imaging sensor 9IR, and facilitates capturing the green light VLG (further the blue light VLB and the red light VLR) with high resolution and capturing the infrared light FLIR clearly. Several examples of specific positioning methods will be described.

<Positioning method 3-1>

In one embodiment, the long-pass filter 8IR may have an optical axial length designed according to an optical path length difference between the green light VLG and the infrared light FLIR so as to position the infrared imaging sensor 9IR at the imaging position of the infrared light FLIR.

<Positioning method 3-2>

In one embodiment, the prism 6 may have optical axial lengths designed according to the optical path length difference described above so as to position the green light imaging sensor 7G at the imaging position of the green light VLG and position the infrared imaging sensor 9IR at the imaging position of the infrared light FLIR. For example, adjusting an optical axial length of the block 611 makes it possible to bring the green light imaging sensor 7G closer to or away from the prism 6. Adjusting the optical axial length of the block 62 makes it possible to bring the infrared imaging sensor 9IR closer to or away from the prism 6.

<Positioning method 3-3>

In one embodiment, the imaging device 1 may include a gap positioned between the prism 6 and at least one of the green light imaging sensor 7G and the infrared imaging sensor 9IR. The gap portion may have optical axial lengths designed according to the optical path length difference described above so as to position the green light imaging sensor 7G at the imaging position of the green light VLG and position the infrared imaging sensor 9IR at the imaging position of the infrared light FLIR. A holding member for providing the gap configured as described above may be used. The gap and the holding member may have similar configurations to the gap and the holding member described in the first embodiment with reference to FIG. 10.

4. Modifications

The disclosed technology is not limited to the above embodiments. Several examples will be described.
The positions of the imaging sensors may be appropriately interchanged. For example, in the first embodiment, the positions of the visible-light imaging sensor 7 and the fluorescence imaging sensor 9 may be interchanged. The characteristic of the dichroic film 65 of the prism 6, the arrangement of the long-pass filter 8, and the like may also be changed according to the interchange. The positions of the visible-light imaging sensor 7 and the fluorescence imaging sensor 9 may be interchanged also in the second embodiment. Furthermore, the positions of the fluorescence imaging sensor 91 and the fluorescence imaging sensor 92 may be interchanged. The characteristic of the dichroic film 66 of the prism 6, the arrangement of the long-pass filter 81 and the long-pass filter 82, and the like may also be changed according to the interchange. The positions of the visible-light imaging sensor 7 and the fluorescence imaging sensor 9 may be interchanged also in the third embodiment. Furthermore, the positions of the green light imaging sensor 7G, the blue light imaging sensor 7B, and the red light imaging sensor 7R may be interchanged. The characteristics of the dichroic film 67, the dichroic film 68, and the dichroic film 69 of the prism 6, the arrangement of the long-pass filter 8IR, and the like may be changed according to the interchange.
As partially described above, there may be a gap (for example, air gap) between the prism 6 and the visible-light imaging sensor 7, between the prism 6 and the long-pass filter 8, or between the long-pass filter 8 and the visible-light imaging sensor 7. Furthermore, the excitation light cut filter 5 may be bonded to the block 61 of the prism 6. There may be an air gap between blocks included in the prism 6.

5. Application examples

The technology according to the present disclosure is applicable to a medical imaging system. The medical imaging system is a medical system using an imaging technology, and is, for example, a medical observation system such as an endoscope system or a microscope system.

<Endoscope system>

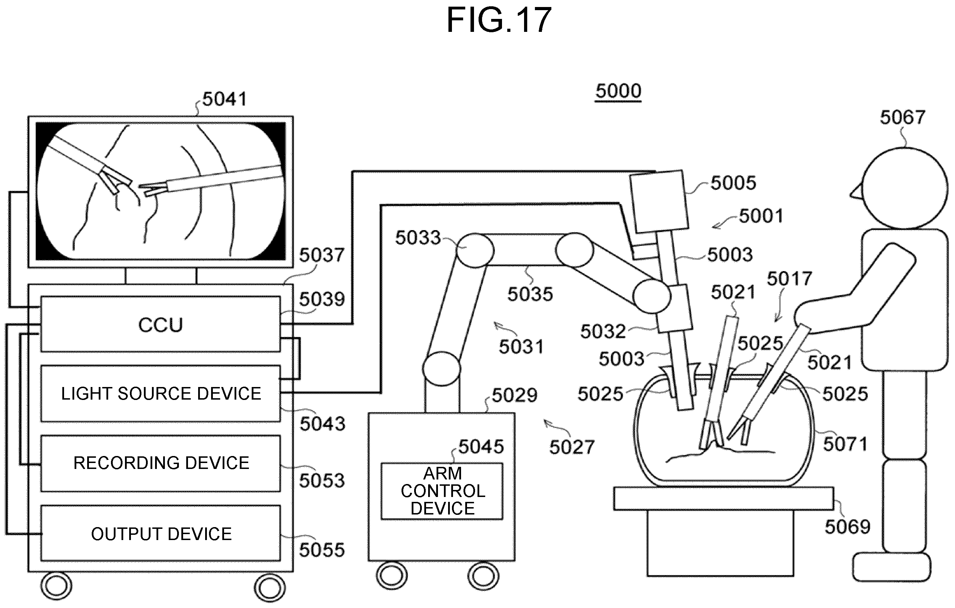

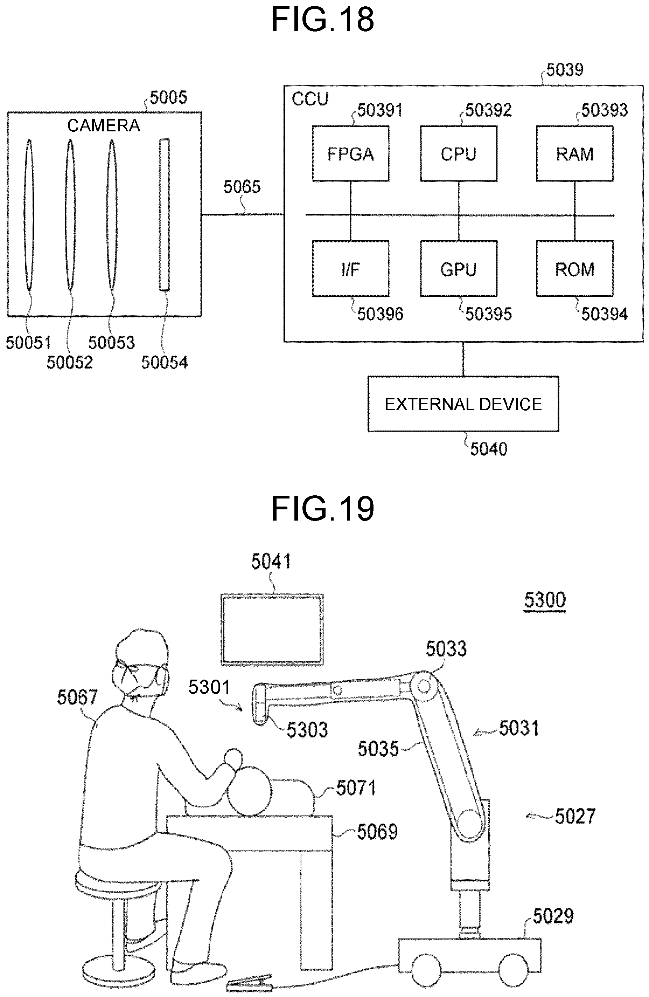

An example of the endoscope system will be described using FIGS. 17 and 18. FIG. 17 is a diagram illustrating an example of a schematic configuration of an endoscope system 5000 to which the technology according to the present disclosure is applicable. FIG. 18 is a diagram illustrating an example of a configuration of an endoscope 5001 and a camera control unit (CCU) 5039. FIG. 17 illustrates a situation where an operator (for example, a doctor) 5067 who is a participant of an operation performs the operation on a patient 5071 on a patient bed 5069 using the endoscope system 5000. As illustrated in FIG. 17, the endoscope system 5000 includes the endoscope 5001 that is a medical imaging device, the CCU 5039, a light source device 5043, a recording device 5053, an output device 5055, and a support device 5027 for supporting the endoscope 5001.
In endoscopic surgery, insertion assisting tools called trocars 5025 are punctured into the patient 5071. Then, a scope 5003 connected to the endoscope 5001 and surgical tools 5021 are inserted into a body of the patient 5071 through the trocars 5025. The surgical tools 5021 include: an energy device such as an electric scalpel; and forceps, for example.
A surgical image that is a medical image in which the inside of the body of the patient 5071 is captured by the endoscope 5001 is displayed on a display device 5041. The operator 5067 performs a procedure on a surgical target using the surgical tools 5021 while viewing the surgical image displayed on the display device 5041. The medical image is not limited to the surgical image, and may be a diagnostic image captured during diagnosis.

<Endoscope>

The endoscope 5001 is an imaging section for capturing the inside of the body of the patient 5071, and is, for example, as illustrated in FIG. 18, a camera including a condensing optical system 50051 for condensing incident light, a zooming optical system 50052 capable of optical zooming by changing a focal length of the imaging section, a focusing optical system 50053 capable of focus adjustment by changing the focal length of the imaging section, and a light receiving sensor 50054. The endoscope 5001 condenses the light through the connected scope 5003 on the light receiving sensor 50054 to generate a pixel signal, and outputs the pixel signal through a transmission system to the CCU 5039. The scope 5003 is an insertion part that includes an objective lens at a distal end and guides the light from the connected light source device 5043 into the body of the patient 5071. The scope 5003 is, for example, a rigid scope for a rigid endoscope and a flexible scope for a flexible endoscope. The scope 5003 may be a direct viewing scope or an oblique viewing scope. The pixel signal only needs to be a signal based on a signal output from a pixel, and is, for example, a raw signal or an image signal. The transmission system connecting the endoscope 5001 to the CCU 5039 may include a memory, and the memory may store parameters related to the endoscope 5001 and the CCU 5039. The memory may be disposed at a connection portion of the transmission system or on a cable. For example, the memory of the transmission system may store the parameters before shipment of the endoscope 5001 or the parameters changed when current is applied, and an operation of the endoscope may be changed based on the parameters read from the memory. A set of the camera and the transmission system may be referred to as an endoscope. The light receiving sensor 50054 is a sensor for converting the received light into the pixel signal, and is, for example, a complementary metal-oxide-semiconductor (CMOS) imaging sensor. The light receiving sensor 50054 is preferably an imaging sensor having a Bayer array capable of color imaging. The light receiving sensor 50054 is also preferably an imaging sensor having a number of pixels corresponding to a resolution of, for example, 4K (3840 horizontal pixels × 2160 vertical pixels), 8K (7680 horizontal pixels × 4320 vertical pixels), or square 4K (3840 or more horizontal pixels × 3840 or more vertical pixels). The light receiving sensor 50054 may be one sensor chip, or a plurality of sensor chips. For example, a prism may be provided to separate the incident light into predetermined wavelength bands, and the wavelength bands may be imaged by different light receiving sensors. A plurality of light receiving sensors may be provided for stereoscopic viewing. The light receiving sensor 50054 may be a sensor having a chip structure including an arithmetic processing circuit for image processing, or may be a sensor for time of flight (ToF). The transmission system is, for example, an optical fiber cable system or a wireless transmission system. The wireless transmission only needs to be capable of transmitting the pixel signal generated by the endoscope 5001, and, for example, the endoscope 5001 may be wirelessly connected to the CCU 5039, or the endoscope 5001 may be connected to the CCU 5039 via a base station in an operating room. At this time, the endoscope 5001 may transmit not only the pixel signal, but also simultaneously information (for example, a processing priority of the pixel signal and/or a synchronization signal) related to the pixel signal. In the endoscope, the scope may be integrated with the camera, and the light receiving sensor may be provided at the distal end of the scope.

<Camera control unit (CCU)>

The CCU 5039 is a control device for controlling the endoscope 5001 and the light source device 5043 connected to the CCU 5039 in an integrated manner, and is, for example, as illustrated in FIG. 18, an image processing device including a field-programmable gate array (FPGA) 50391, a central processing unit (CPU) 50392, a random access memory 50393, a read-only memory (ROM) 50394, a graphics processing unit (GPU) 50395, and an interface (I/F) 50396. The CCU 5039 may control the display device 5041, the recording device 5053, and the output device 5055 connected to the CCU 5039 in an integrated manner. The CCU 5039 controls, for example, irradiation timing, irradiation intensity, and a type of an irradiation light source of the light source device 5043. The CCU 5039 also performs image processing, such as development processing (for example, demosaic processing) and correction processing, on the pixel signal output from the endoscope 5001, and outputs the processed image signal (for example, an image) to an external device such as the display device 5041. The CCU 5039 also transmits a control signal to the endoscope 5001 to control driving of the endoscope 5001. The control signal is information on an imaging condition such as a magnification or the focal length of the imaging section. The CCU 5039 may have a function to down-convert the image, and may be configured to be capable of simultaneously outputting a higher-resolution (for example, 4K) image to the display device 5041 and a lower-resolution (for example, high-definition (HD)) image to the recording device 5053.

The CCU 5039 may be connected to external equipment (such as a recording device, a display device, an output device, and a support device) via an IP converter for converting the signal into a predetermined communication protocol (such as the Internet Protocol (IP)). The connection between the IP converter and the external equipment may be established using a wired network, or a part or the whole of the network may be established using a wireless network. For example, the IP converter on the CCU 5039 side may have a wireless communication function, and may transmit the received image to an IP switcher or an output side IP converter via a wireless communication network, such as the fifth-generation mobile communication system (5G) or the sixth-generation mobile communication system (6G).

<Light source device>

The light source device 5043 is a device capable of emitting the light having predetermined wavelength bands, and includes, for example, a plurality of light sources and a light source optical system for guiding the light of the light sources. The light sources are, for example, xenon lamps, light-emitting diode (LED) light sources, or laser diode (LD) light sources. The light source device 5043 includes, for example, the LED light sources corresponding to three respective primary colors of red (R), green (G), and blue (B), and controls output intensity and output timing of each of the light sources to emit white light. The light source device 5043 may include a light source capable of emitting special light used for special light observation, in addition to the light sources for emitting normal light for normal light observation. The special light is light having a predetermined wavelength band different from that of the normal light being light for the normal light observation, and is, for example, near-infrared light (light having a wavelength of 760 nm or longer), infrared light, blue light, or ultraviolet light. The normal light is, for example, the white light or green light. In narrow band imaging that is a kind of special light observation, blue light and green light are alternately emitted, and thus the narrow band imaging can image a predetermined tissue such as a blood vessel in a mucosal surface at high contrast using wavelength dependence of light absorption in the tissue of the body. In fluorescence observation that is a kind of special light observation, excitation light is emitted for exciting an agent injected into the tissue of the body, and fluorescence emitted by the tissue of the body or the agent as a label is received to obtain a fluorescent image, and thus the fluorescence observation can facilitate the operator to view, for example, the tissue of the body that is difficult to be viewed by the operator with the normal light. For example, in fluorescence observation using the infrared light, the infrared light having an excitation wavelength band is emitted to an agent, such as indocyanine green (ICG), injected into the tissue of the body, and the fluorescence light from the agent is received, whereby the fluorescence observation can facilitate viewing of a structure and an affected part of the tissue of the body. In the fluorescence observation, an agent (such as 5-aminolevulinic acid (5-ALA)) may be used that emits fluorescence in a red wavelength band by being excited by the special light in a blue wavelength band. The type of the irradiation light of the light source device 5043 is set by control of the CCU 5039. The CCU 5039 may have a mode of controlling the light source device 5043 and the endoscope 5001 to alternately perform the normal light observation and the special light observation. At this time, information based on a pixel signal obtained by the special light observation is preferably superimposed on a pixel signal obtained by the normal light observation. The special light observation may be an infrared light observation to observe a site inside the surface of an organ and a multi-spectrum observation utilizing hyperspectral spectroscopy. A photodynamic therapy may be incorporated.

<Recording device>

The recording device 5053 is a device for recording the pixel signal (for example, an image) acquired from the CCU 5039, and is, for example, a recorder. The recording device 5053 records an image acquired from the CCU 5039 in a hard disk drive (HDD), a Super Density Disc (SDD), and/or an optical disc. The recording device 5053 may be connected to a network in a hospital to be accessible from equipment outside the operating room. The recording device 5053 may have a down-convert function or an up-convert function.

<Display device>

The display device 5041 is a device capable of displaying the image, and is, for example, a display monitor. The display device 5041 displays a display image based on the pixel signal acquired from the CCU 5039. The display device 5041 may include a camera and a microphone to function as an input device that allows instruction input through gaze recognition, voice recognition, and gesture.

<Output device>

The output device 5055 is a device for outputting the information acquired from the CCU 5039, and is, for example, a printer. The output device 5055 prints, for example, a print image based on the pixel signal acquired from the CCU 5039 on a sheet of paper.

<Support device>

The support device 5027 is an articulated arm including a base 5029 including an arm control device 5045, an arm 5031 extending from the base 5029, and a holding part 5032 mounted at a distal end of the arm 5031. The arm control device 5045 includes a processor such as a CPU, and operates according to a predetermined computer program to control driving of the arm 5031. The support device 5027 uses the arm control device 5045 to control parameters including, for example, lengths of links 5035 constituting the arm 5031 and rotation angles and torque of joints 5033 so as to control, for example, the position and attitude of the endoscope 5001 held by the holding part 5032. This control can change the position or attitude of the endoscope 5001 to a desired position or attitude, makes it possible to insert the scope 5003 into the patient 5071, and can change the observed area in the body. The support device 5027 functions as an endoscope support arm for supporting the endoscope 5001 during the operation. Thus, the support device 5027 can play a role of a scopist who is an assistant holding the endoscope 5001. The support device 5027 may be a device for holding a microscope device 5301 to be described later, and can be called a medical support arm. The support device 5027 may be controlled using an autonomous control method by the arm control device 5045, or may be controlled using a control method in which the arm control device 5045 performs the control based on input of a user. The control method may be, for example, a master-slave method in which the support device 5027 serving as a slave device (replica device) that is a patient cart is controlled based on a movement of a master device (primary device) that is an operator console at a hand of the user. The support device 5027 may be remotely controllable from outside the operating room.

The example of the endoscope system 5000 to which the technology according to the present disclosure is applicable has been described above. For example, the technology according to the present disclosure may be applied to a microscope system.

<Microscope system>

FIG. 19 is a diagram illustrating an example of a schematic configuration of a microscopic surgery system to which the technology according to the present disclosure is applicable. In the following description, the same components as those of the endoscope system 5000 will be denoted by the same reference numerals, and the description thereof will not be repeated.

FIG. 19 schematically illustrates a situation where the operator 5067 performs an operation on the patient 5071 on the patient bed 5069 using a microscopic surgery system 5300. For the sake of simplicity, FIG. 19 does not illustrate a cart 5037 among the components of the microscopic surgery system 5300, and illustrates the microscope device 5301 instead of the endoscope 5001 in a simplified manner. The microscope device 5301 may refer to a microscope 5303 provided at the distal end of the links 5035, or may refer to the overall configuration including the microscope 5303 and the support device 5027.

As illustrated in FIG. 19, during the operation, the microscopic surgery system 5300 is used to display an image of a surgical site captured by the microscope device 5301 in a magnified manner on the display device 5041 installed in the operating room. The display device 5041 is installed in a position facing the operator 5067, and the operator 5067 performs various procedures, such as excision of an affected part, on the surgical site while observing the state of the surgical site using the image displayed on the display device 5041. The microscopic surgery system is used in, for example, ophthalmic operation and neurosurgical operation.

The respective examples of the endoscope system 5000 and the microscopic surgery system 5300 to which the technology according to the present disclosure is applicable have been described above. Systems to which the technology according to the present disclosure is applicable are not limited to such examples. For example, the support device 5027 can support, at the distal end thereof, another observation device or another surgical tool instead of the endoscope 5001 or the microscope 5303. Examples of the other applicable observation device include forceps, tweezers, a pneumoperitoneum tube for pneumoperitoneum, and an energy treatment tool for incising a tissue or sealing a blood vessel by cauterization. By using the support device to support the observation device or the surgical tool described above, the position thereof can be more stably fixed and the load of the medical staff can be lower than in a case where the medical staff manually supports the observation device or the surgical tool. The technology according to the present disclosure may be applied to a support device for supporting such a component other than the microscope.

The imaging device 1 according to each embodiment which has been described above is suitably applicable to the endoscope 5001 and the microscope device 5301 of the above-described configurations. Specifically, the target S to be observed is a surgical site. The light source 2 can constitute the light source device 5043. The optical diagnosis assembly 3 can partially constitute the endoscope 5001 or the microscope device 5301. The imaging lens 4, the excitation light cut filter 5, the prism 6, the visible-light imaging sensor 7, the long-pass filter 8, the fluorescence imaging sensor 9, and the like can function as the imaging section. Use of the imaging device 1 makes it possible to capture the visible light (normal light) with high resolution and capture the fluorescence (special light) clearly.

6. Examples of effects

For example, the technology described above is specified as follows. One aspect of the disclosed technology is an imaging device 1. As described with reference to FIGS. 1 to 9 and the like, the imaging device 1 includes the prism 6 that separates the visible light VL and the fluorescence FL included in the light L from the target S to be observed, the visible-light imaging sensor 7 that captures the visible light VL separated by the prism 6, and the fluorescence imaging sensor 9 that captures the fluorescence FL separated by the prism 6. The ratio of the pixel spacing P2 in the fluorescence imaging sensor 9 to the pixel spacing P1 in the visible-light imaging sensor 7 is 1 or more and less than 5 ($1 \leq$ (P2/P1) < 5). Designing the pixel spacing P1 in the visible-light imaging sensor 7 and the pixel spacing P2 in the fluorescence imaging sensor 9 in this manner makes it possible to capture the visible light VL with high resolution and capture the fluorescence FL clearly.

As described with reference to FIGS. 1 and 6 and the like, the imaging device 1 may include the long-pass filter 8 that is provided between the prism 6 and the fluorescence imaging sensor 9 to transmit the fluorescence FL. This configuration makes it possible to prevent input of the unnecessary visible light VL into the fluorescence imaging sensor 9.

As described with reference to FIGS. 1, 3, and 7 and the like, the ratio of the valid pixel area diagonal length D2 of the fluorescence imaging sensor 9 to the valid pixel area diagonal length D1 of the visible-light imaging sensor 7 may be larger than 0.7 and less than 1.4 ($0.7 \leq$ (D2/D1) < 1.4). For example, in this manner, the fluorescence FL can be received with high sensitivity and captured clearly.

As described with reference to FIG. 1 and the like, a configuration may be provided in which the light L after passing through the imaging lens 4 is input into the prism 6, the optical path lengths of the visible light VL and the fluorescence FL, from the input into the prism 6 to imaging, are different from each other, the visible-light imaging sensor 7 is directly or indirectly fixed to the prism 6 so as to be located at the imaging position of the visible light VL, and the fluorescence imaging sensor 9 is directly or indirectly fixed to the prism 6 so as to be located at the imaging position of the fluorescence FL. This configuration only requires focusing of the visible-light imaging sensor 7 to focus the fluorescence imaging sensor 9, and facilitates capturing the visible light VL with high resolution and capturing the fluorescence FL clearly.

As described with reference to FIGS. 1 and 10 and the like, the long-pass filter 8 may have the optical axial length designed according to the optical path length difference between the optical path lengths of the visible light VL and the fluorescence FL so as to position the fluorescence imaging sensor 9 at the imaging position of the fluorescence FL. The prism 6 may have the optical axial lengths designed according to the optical path length difference between the optical path lengths of the visible light VL and the fluorescence FL so as to position the visible-light imaging sensor 7 at the imaging position of the visible light VL and position the fluorescence imaging sensor 9 at the imaging position of the fluorescence FL. The imaging device 1 includes the gap G that is positioned between the prism 6 and at least one of the visible-light imaging sensor 7 and the fluorescence imaging sensor 9, in which the gap G may have optical axial lengths designed according to the optical path length difference between the optical path lengths of the visible light VL and the fluorescence FL so as to position the visible-light imaging sensor 7 at the imaging position of the visible light VL and position the fluorescence imaging sensor 9 at the imaging position of the fluorescence FL. For example, the visible-light imaging sensor 7 and the fluorescence imaging sensor 9 can be positioned in this manner.

As described with reference to FIGS. 11 and 12 and the like, the fluorescence FL includes the fluorescence FL1 (first fluorescence) and the fluorescence FL2 (second fluorescence), the prism 6 separates the visible light VL, the fluorescence FL1, and the fluorescence FL2 included in the light L from the target S to be observed, and the fluorescence imaging sensor 9 includes the fluorescence imaging sensor 91 (first fluorescence imaging sensor) that captures the fluorescence FL1 separated by the prism 6 and the fluorescence imaging sensor 92 (second fluorescence imaging sensor) that captures the fluorescence FL2 separated by the prism 6, in which both of the ratio of the pixel spacing P4 in the fluorescence imaging sensor 91 to the pixel spacing P3 in the visible-light imaging sensor 7 and the ratio of the pixel spacing P5 in the fluorescence imaging sensor 92 to the pixel spacing P3 in the visible-light imaging sensor 7 may be 1 or more and less than 5 ($1 \leq$ (P4/P3) < 5 and $1 \leq$ (P5/P3) < 5). This configuration makes it possible to capture the visible light VL with high resolution and capture the fluorescence FL1 and the fluorescence FL2 clearly.

As described with reference to FIG. 11 and the like, both of a ratio of the valid pixel area diagonal length D4 of the fluorescence imaging sensor 91 to the valid pixel area diagonal length D3 of the visible-light imaging sensor 7 and a ratio of the valid pixel area diagonal length D5 of the fluorescence imaging sensor 92 to the valid pixel area diagonal length D3 of the visible-light imaging sensor 7 may be larger than 0.7 and smaller than 1.4 ($0.7 \leq$ (D4/D3) < 1.4 and $0.7 \leq$ (D5/D3) < 1.4). For example, in this manner, the fluorescence FL1 and the fluorescence FL2 can be received with high sensitivity and captured clearly.

As described with reference to FIG. 11 and the like, a configuration may be provided in which the optical path lengths of the visible light VL, the fluorescence FL1, and the fluorescence FL2, from the input into the prism 6 to the imaging, are different from each other, the visible-light imaging sensor 7 is directly or indirectly fixed to the prism 6 so as to be located at the imaging position of the visible light VL, the fluorescence imaging sensor 91 is directly or indirectly fixed to the prism 6 so as to be located at the imaging position of the fluorescence FL1, and the fluorescence imaging sensor 92 may be directly or indirectly fixed to the prism 6 so as to be located at the imaging position of the fluorescence FL2. This configuration only requires focusing of the visible-light imaging sensor 7 to focus the fluorescence imaging sensor 91 and the fluorescence imaging sensor 92, and facilitates capturing the visible light VL with high resolution and capturing the fluorescence FL1 and the fluorescence FL2 clearly.

As described with reference to FIGS. 10 and 11 and the like, the imaging device 1 includes the long-pass filter 81 (a first long-pass filter) that is provided between the prism 6 and the fluorescence imaging sensor 91 to transmit the fluorescence FL1, and the long-pass filter 82 (a second long-pass filter) that is provided between the prism 6 and the fluorescence imaging sensor 92 to transmit the fluorescence FL2, in which the long-pass filter 81 may have the optical axial length designed according to the optical path length difference between the optical path lengths of the visible light VL and the fluorescence FL1 so as to position the fluorescence imaging sensor 91 at the imaging position of the fluorescence FL1, and the long-pass filter 82 may have the optical axial length designed according to the optical path length difference between the optical path lengths of the visible light VL and the fluorescence FL2 so as to position the fluorescence imaging sensor 92 at the imaging position of the fluorescence FL2. The prism 6 may have the optical axial lengths designed according to the optical path length difference between the optical path lengths of the visible light VL, the fluorescence FL1, and the fluorescence FL2 so as to position the visible-light imaging sensor 7 at the imaging position of the visible light VL, position the fluorescence imaging sensor 91 at the imaging position of the fluorescence FL1, and position the fluorescence imaging sensor 92 at the imaging position of the fluorescence FL2. The imaging device 1 includes the gap G that is positioned between the prism 6 and at least one of the visible-light imaging sensor 7, the fluorescence imaging sensor 91, and the fluorescence imaging sensor 92, in which the gap G may have the optical axial lengths designed according to the optical path length difference between the optical path lengths of the visible light VL, the fluorescence FL1, and the fluorescence FL2 so as to position the visible-light imaging sensor 7 at the imaging position of the visible light VL, position the fluorescence imaging sensor 91 at the imaging position of the fluorescence FL1, and position the fluorescence imaging sensor 92 at the imaging position of the fluorescence FL2. For example, in this manner, the visible-light imaging sensor 7, the fluorescence imaging sensor 91, and the fluorescence imaging sensor 92 can be positioned.

As described with reference to FIGS. 13 to 16 and the like, the visible light VL includes the green light VLG, the blue light VLB, and the red light VLR, the fluorescence FL includes the infrared light FLIR, the prism 6 separates the green light VLG, the blue light VLB, the red light VLR, and the infrared light FLIR included in the light L from the target S to be observed, the visible-light imaging sensor 7 includes the green light imaging sensor 7G that captures the green light VLG separated by the prism 6, the blue light imaging sensor 7B that captures the blue light VLB separated by the prism 6, and the red light imaging sensor 7R that captures the red light VLR separated by the prism 6, and the fluorescence imaging sensor 9 includes the infrared imaging sensor 9IR that images the infrared light FLIR separated by the prism 6, in which the ratio of the pixel spacing P7 in the infrared imaging sensor 9IR to the pixel spacing P6 in the green light imaging sensor 7G may be 1 or more and less than 5 ($1 \leq (P7/P6) < 5$). This configuration makes it possible to capture the green light VLG, the blue light VLB, and the red light VLR with high resolution and capture the infrared light FLIR clearly.

As described with reference to FIG. 13 and the like, the ratio of the valid pixel area diagonal length D7 of the infrared imaging sensor 9IR to the valid pixel area diagonal length D6 of the green light imaging sensor 7G may be larger than 0.7 and less than 1.4 ($0.7 \leq (D7/D6) < 1.4$). For example, in this manner, the infrared light FLIR can be received with high sensitivity and captured clearly.

As described with reference to FIG. 13 and the like, a configuration may be provided in which the optical path lengths of the green light VLG and the infrared light FLIR, from the input into the prism 6 to the imaging, are different from each other, the green light imaging sensor 7G is directly or indirectly fixed to the prism 6 so as to be located at the imaging position of the green light VLG, and the infrared imaging sensor 9IR is directly or indirectly fixed to the prism 6 so as to be located at the imaging position of the infrared light FLIR. This configuration only requires focusing of the green light imaging sensor 7G to focus the infrared imaging sensor 9IR, and facilitates capturing the green light VLG with high resolution and capturing the infrared light FLIR clearly.

As described with reference to FIGS. 10 and 13 and the like, the imaging device 1 includes the long-pass filter 8IR that is provided between the prism 6 and the infrared imaging sensor 9IR to transmit the infrared light FLIR, in which the long-pass filter 8IR may have the optical axial length designed according to the optical path length difference between the optical path lengths of the green light VLG and the infrared light FLIR so as to position the infrared imaging sensor 9IR at the imaging position of the infrared light FLIR. The prism 6 may have the optical axial lengths designed according to the optical path length difference between the optical path lengths of the green light VLG and the infrared light FLIR so as to position the green light imaging sensor 7G at the imaging position of the green light VLG and position the infrared imaging sensor 9IR at the imaging position of the infrared light FLIR. The imaging device 1 includes the gap G that is positioned between the prism 6 and at least one of the green light imaging sensor 7G and the infrared imaging sensor 9IR, in which the gap G may have the optical axial lengths designed according to the optical path length difference between the optical path lengths of the green light VLG and the infrared light FLIR so as to position the green light imaging sensor 7G at the imaging position of the green light VLG and position the infrared imaging sensor 9IR at the imaging position of the infrared light FLIR. For example, in this manner, the green light imaging sensor 7G and the infrared imaging sensor 9IR can be positioned.

The medical observation system (for example, the endoscope system 5000, the microscopic surgery system 5300, and the like) described with reference to FIGS. 17 to 19 is also one aspect of the disclosed technology. The medical observation system includes the imaging device 1 that captures the surgical site. The imaging device 1 is configured as described above. In medical observation, it is possible to capture the visible light VL (normal light) with high resolution and capture the fluorescence FL (special light) clearly.

Note that the effects described in the present disclosure are merely examples and are not limited to the disclosed contents. There may be other effects.

Although embodiments of the present disclosure have been described above, the technical scope of the present disclosure is not limited to the above embodiments as they are and various changes can be made without departing from the gist of the present disclosure. Furthermore, the components in different embodiments and modifications may be combined as appropriate.

Note that the present technology can also have the following configurations.

(1) An imaging device comprising:

a prism that separates visible light and fluorescence included in light from a target to be observed;
a visible-light imaging sensor that captures the visible light separated by the prism; and
a fluorescence imaging sensor that captures the fluorescence separated by the prism, wherein
a ratio of a pixel spacing in the fluorescence imaging sensor to a pixel spacing in the visible-light imaging sensor is 1 or more and less than 5.

(2) The imaging device according to (1), comprising
a long-pass filter that is provided between the prism and the fluorescence imaging sensor to transmit the fluorescence.
(3) The imaging device according to (1) or (2), wherein
a ratio of a valid pixel area diagonal length of the fluorescence imaging sensor to a valid pixel area diagonal length of the visible-light imaging sensor is larger than 0.7 and less than 1.4.
(4) The imaging device according to any one of (1) to (3), wherein

light after passing through an imaging lens is input into the prism,
optical path lengths of the visible light and the fluorescence, from the input into the prism to imaging, are different from each other,
the visible-light imaging sensor is directly or indirectly fixed to the prism to be located at an imaging position of the visible light, and
the fluorescence imaging sensor is directly or indirectly fixed to the prism to be located at an imaging position of the fluorescence.

(5) The imaging device according to (4), comprising

a long-pass filter that is provided between the prism and the fluorescence imaging sensor to transmit the fluorescence, wherein
the long-pass filter has an optical axial length designed according to an optical path length difference between the optical path lengths of the visible light and the fluorescence so as to position the fluorescence imaging sensor at the imaging position of the fluorescence.

(6) The imaging device according to (4) or (5), wherein
the prism has optical axial lengths designed according to an optical path length difference between the optical path lengths of the visible light and the fluorescence so as to position the visible-light imaging sensor at the imaging position of the visible light and position the fluorescence imaging sensor at the imaging position of the fluorescence.
(7) The imaging device according to any one of (4) to (6), comprising

a gap that is positioned between the prism and at least one of the visible-light imaging sensor and the fluorescence imaging sensor, wherein
the gap has optical axial lengths designed according to an optical path length difference between the optical path lengths of the visible light and the fluorescence so as to position the visible-light imaging sensor at the imaging position of the visible light and position the fluorescence imaging sensor at the imaging position of the fluorescence.

(8) The imaging device according to any one of (1) to (7), wherein

the fluorescence includes first fluorescence and second fluorescence,
the prism separates the visible light, the first fluorescence, and the second fluorescence included in the light from the target to be observed,
the fluorescence imaging sensor includes:

a first fluorescence imaging sensor that captures the first fluorescence separated by the prism; and

a second fluorescence imaging sensor that captures the second fluorescence separated by the prism, and both of a ratio of a pixel spacing in the first fluorescence imaging sensor to the pixel spacing in the visible-light imaging sensor and a ratio of a pixel spacing in the second fluorescence imaging sensor to the pixel spacing in the visible-light imaging sensor are 1 or more and less than 5.

(9) The imaging device according to (8), wherein
both of a ratio of a valid pixel area diagonal length of the first fluorescence imaging sensor to a valid pixel area diagonal length of the visible-light imaging sensor and a ratio of a valid pixel area diagonal length of the second fluorescence imaging sensor to the valid pixel area diagonal length of the visible-light imaging sensor are larger than 0.7 and less than 1.4.

(10) The imaging device according to (8) or (9), wherein

light after passing through an imaging lens is input into the prism,
optical path lengths of the visible light, the first fluorescence, and the second fluorescence, from the input into the prism to imaging, are different from each other,
the visible-light imaging sensor is directly or indirectly arranged at the prism so as to be located at an imaging position of the visible light,
the first fluorescence imaging sensor is directly or indirectly fixed to the prism so as to be located at an imaging position of the first fluorescence, and
the second fluorescence imaging sensor is directly or indirectly fixed to the prism so as to be located at an imaging position of the second fluorescence.

(11) The imaging device according to (10), comprising:

a first long-pass filter that is provided between the prism and the first fluorescence imaging sensor to transmit the first fluorescence; and
a second long-pass filter that is provided between the prism and the second fluorescence imaging sensor to transmit the second fluorescence, wherein
the first long-pass filter has an optical axial length designed according to an optical path length difference between the optical path lengths of the visible light and the first fluorescence so as to position the first fluorescence imaging sensor at the imaging position of the first fluorescence, and
the second long-pass filter has an optical axial length designed according to an optical path length difference between the optical path lengths of the visible light and the second fluorescence so as to position the second fluorescence imaging sensor at the imaging position of the second fluorescence.

(12) The imaging device according to (10) or (11), wherein
the prism has optical axial lengths designed according to an optical path length difference between the optical path lengths of the visible light, the first fluorescence, and the second fluorescence so as to position the visible-light imaging sensor at the imaging position of the visible light, position the first fluorescence imaging sensor at the imaging position of the first fluorescence, and position the second fluorescence imaging sensor at the imaging position of the second fluorescence.

(13) The imaging device according to any one of (10) to (12), comprising

a gap that is positioned between the prism and at least one of the visible-light imaging sensor, the first fluorescence imaging sensor, and the second fluorescence imaging sensor, wherein
the gap has optical axial lengths designed according to an optical path length difference between the optical path lengths of the visible light, the first fluorescence, and the second fluorescence so as to position the visible-light imaging sensor at the imaging position of the visible light, position the first fluorescence imaging sensor at the imaging position of the first fluorescence, and position the second fluorescence imaging sensor at the imaging position of the second fluorescence.

(14) The imaging device according to any one of (1) to (13), wherein

the visible light includes green light, blue light, and red light,
the fluorescence includes infrared light,
the prism separates the green light, the blue light, the red light, and the infrared light included in the light from the target to be observed,
the visible-light imaging sensor includes:

a green light imaging sensor that captures the green light separated by the prism;

a blue light imaging sensor that captures the blue light separated by the prism; and

a red light imaging sensor that captures the red light separated by the prism,

the fluorescence imaging sensor includes an infrared imaging sensor that captures the infrared light separated by the prism, and

a ratio of a pixel spacing in the infrared imaging sensor to a pixel spacing in the green light imaging sensor is 1 or more and less than 5.

(15) The imaging device according to (14), wherein
a ratio of a valid pixel area diagonal length of the infrared imaging sensor to a valid pixel area diagonal length of the green light imaging sensor is larger than 0.7 and less than 1.4.
(16) The imaging device according to (14) or (15), wherein

light after passing through an imaging lens is input into the prism,

optical path lengths of the green light and the infrared light, from the input into the prism to imaging, are different from each other,

the green light imaging sensor is directly or indirectly fixed to the prism so as to be located at an imaging position of the green light, and

the infrared imaging sensor is directly or indirectly fixed to the prism so as to be located at an imaging position of the infrared light.

(17) The imaging device according to (16), comprising

a long-pass filter that is provided between the prism and the infrared imaging sensor to transmit the infrared light, wherein

the long-pass filter has an optical axial length designed according to an optical path length difference between the optical path lengths of the green light and the infrared light so as to position the infrared imaging sensor at the imaging position of the infrared light.

(18) The imaging device according to (16) or (17), wherein
the prism has optical axial lengths designed according to an optical path length difference between the optical path lengths of the green light and the infrared light so as to position the green light imaging sensor at the imaging position of the green light and position the infrared imaging sensor at the imaging position of the infrared light.
(19) The imaging device according to any one of (16) to (18), comprising

a gap that is positioned between the prism and at least one of the green light imaging sensor and the infrared imaging sensor, wherein

the gap has optical axial lengths designed according to an optical path length difference between the optical path lengths of the green light and the infrared light so as to position the green light imaging sensor at the imaging position of the green light and position the infrared imaging sensor at the imaging position of the infrared light.

(20) A medical observation system comprising

an imaging device that captures a surgical site, wherein
the imaging device includes:

a prism that separates visible light and fluorescence included in light from the surgical site;

a visible-light imaging sensor that captures the visible light separated by the prism; and

a fluorescence imaging sensor that captures the fluorescence separated by the prism, and

a ratio of a pixel spacing in the fluorescence imaging sensor to a pixel spacing in the visible-light imaging sensor is 1 or more and less than 5.

Reference Signs List

1 IMAGING DEVICE
2 LIGHT SOURCE

21 WHITE LIGHT SOURCE
22 EXCITATION LIGHT SOURCE
3 OPTICAL DIAGNOSIS ASSEMBLY
4 IMAGING LENS
4a INPUT SURFACE
4b OUTPUT SURFACE
5 EXCITATION LIGHT CUT FILTER
5a INPUT SURFACE
5b OUTPUT SURFACE
6 PRISM
6a INPUT SURFACE
6b1 OUTPUT SURFACE
6b2 OUTPUT SURFACE
6b21 OUTPUT SURFACE
6b22 OUTPUT SURFACE
6bB OUTPUT SURFACE
6bG OUTPUT SURFACE
6bR OUTPUT SURFACE
6bIR OUTPUT SURFACE
61 BLOCK
611 BLOCK
612 BLOCK
613 BLOCK
62 BLOCK
621 BLOCK
622 BLOCK
65 DICHROIC FILM
66 DICHROIC FILM
67 DICHROIC FILM
68 DICHROIC FILM
69 DICHROIC FILM
7 VISIBLE-LIGHT IMAGING SENSOR
7a LIGHT RECEIVING SURFACE
7p PIXEL
7a1 PIXEL AREA
7a2 VALID PIXEL AREA
7B BLUE LIGHT IMAGING SENSOR
7G GREEN LIGHT IMAGING SENSOR
7R RED LIGHT IMAGING SENSOR
8 LONG-PASS FILTER
8a INPUT SURFACE
8b OUTPUT SURFACE
81 LONG-PASS FILTER
82 LONG-PASS FILTER
8IR LONG-PASS FILTER
9 FLUORESCENCE IMAGING SENSOR
9a LIGHT RECEIVING SURFACE
9p PIXEL
9a1 PIXEL AREA
9a2 VALID PIXEL AREA
91 FLUORESCENCE IMAGING SENSOR
92 FLUORESCENCE IMAGING SENSOR
9IR INFRARED IMAGING SENSOR
10 HOLDING MEMBER
101 BASE PLATE
102 HOLDING ROD
102a ONE END PORTION
102b OTHER END PORTION

5000 ENDOSCOPE SYSTEM (MEDICAL OBSERVATION SYSTEM)
5300 MICROSCOPIC SURGERY SYSTEM (MEDICAL OBSERVATION SYSTEM)
D1 VALID PIXEL AREA DIAGONAL LENGTH
D2 VALID PIXEL AREA DIAGONAL LENGTH
D3 VALID PIXEL AREA DIAGONAL LENGTH
D4 VALID PIXEL AREA DIAGONAL LENGTH
D5 VALID PIXEL AREA DIAGONAL LENGTH
D6 VALID PIXEL AREA DIAGONAL LENGTH
D7 VALID PIXEL AREA DIAGONAL LENGTH
G GAP
P1 PIXEL SPACING
P2 PIXEL SPACING
P3 PIXEL SPACING
P4 PIXEL SPACING
P5 PIXEL SPACING
P6 PIXEL SPACING
P7 PIXEL SPACING
S TARGET TO BE OBSERVED
L LIGHT
SL LIGHT
VL VISIBLE LIGHT
VLB BLUE LIGHT
VLG GREEN LIGHT
VLR RED LIGHT
FL FLUORESCENCE
FL1 FLUORESCENCE
FL2 FLUORESCENCE
FLIR INFRARED LIGHT
WL WHITE LIGHT
EL EXCITATION LIGHT
W1 PIXEL SIZE
W2 PIXEL SIZE

**Claims**

1. An imaging device comprising:

   a prism that separates visible light and fluorescence included in light from a target to be observed;
   a visible-light imaging sensor that captures the visible light separated by the prism; and
   a fluorescence imaging sensor that captures the fluorescence separated by the prism, wherein
   a ratio of a pixel spacing in the fluorescence imaging sensor to a pixel spacing in the visible-light imaging sensor is
   1 or more and less than 5.

2. The imaging device according to claim 1, comprising
   a long-pass filter that is provided between the prism and the fluorescence imaging sensor to transmit the fluorescence.

3. The imaging device according to claim 1, wherein
   a ratio of a valid pixel area diagonal length of the fluorescence imaging sensor to a valid pixel area diagonal length of
   the visible-light imaging sensor is larger than 0.7 and less than 1.4.

4. The imaging device according to claim 1, wherein

   light after passing through an imaging lens is input into the prism,
   optical path lengths of the visible light and the fluorescence, from the input into the prism to imaging, are different
   from each other,
   the visible-light imaging sensor is directly or indirectly fixed to the prism to be located at an imaging position of the
   visible light, and

the fluorescence imaging sensor is directly or indirectly fixed to the prism to be located at an imaging position of the fluorescence.

5. The imaging device according to claim 4, comprising

a long-pass filter that is provided between the prism and the fluorescence imaging sensor to transmit the fluorescence, wherein
the long-pass filter has an optical axial length designed according to an optical path length difference between the optical path lengths of the visible light and the fluorescence so as to position the fluorescence imaging sensor at the imaging position of the fluorescence.

6. The imaging device according to claim 4, wherein
the prism has optical axial lengths designed according to an optical path length difference between the optical path lengths of the visible light and the fluorescence so as to position the visible-light imaging sensor at the imaging position of the visible light and position the fluorescence imaging sensor at the imaging position of the fluorescence.

7. The imaging device according to claim 4, comprising

a gap that is positioned between the prism and at least one of the visible-light imaging sensor and the fluorescence imaging sensor, wherein
the gap has optical axial lengths designed according to an optical path length difference between the optical path lengths of the visible light and the fluorescence so as to position the visible-light imaging sensor at the imaging position of the visible light and position the fluorescence imaging sensor at the imaging position of the fluorescence.

8. The imaging device according to claim 1, wherein

the fluorescence includes first fluorescence and second fluorescence,
the prism separates the visible light, the first fluorescence, and the second fluorescence included in the light from the target to be observed,
the fluorescence imaging sensor includes:

a first fluorescence imaging sensor that captures the first fluorescence separated by the prism; and
a second fluorescence imaging sensor that captures the second fluorescence separated by the prism, and
both of a ratio of a pixel spacing in the first fluorescence imaging sensor to the pixel spacing in the visible-light imaging sensor and a ratio of a pixel spacing in the second fluorescence imaging sensor to the pixel spacing in the visible-light imaging sensor are 1 or more and less than 5.

9. The imaging device according to claim 8, wherein
both of a ratio of a valid pixel area diagonal length of the first fluorescence imaging sensor to a valid pixel area diagonal length of the visible-light imaging sensor and a ratio of a valid pixel area diagonal length of the second fluorescence imaging sensor to the valid pixel area diagonal length of the visible-light imaging sensor are larger than 0.7 and less than 1.4.

10. The imaging device according to claim 8, wherein

light after passing through an imaging lens is input into the prism,
optical path lengths of the visible light, the first fluorescence, and the second fluorescence, from the input into the prism to imaging, are different from each other,
the visible-light imaging sensor is directly or indirectly arranged at the prism so as to be located at an imaging position of the visible light,
the first fluorescence imaging sensor is directly or indirectly fixed to the prism so as to be located at an imaging position of the first fluorescence, and
the second fluorescence imaging sensor is directly or indirectly fixed to the prism so as to be located at an imaging position of the second fluorescence.

11. The imaging device according to claim 10, comprising:

a first long-pass filter that is provided between the prism and the first fluorescence imaging sensor to transmit the first fluorescence; and

a second long-pass filter that is provided between the prism and the second fluorescence imaging sensor to transmit the second fluorescence, wherein

the first long-pass filter has an optical axial length designed according to an optical path length difference between the optical path lengths of the visible light and the first fluorescence so as to position the first fluorescence imaging sensor at the imaging position of the first fluorescence, and

the second long-pass filter has an optical axial length designed according to an optical path length difference between the optical path lengths of the visible light and the second fluorescence so as to position the second fluorescence imaging sensor at the imaging position of the second fluorescence.

12. The imaging device according to claim 10, wherein

the prism has optical axial lengths designed according to an optical path length difference between the optical path lengths of the visible light, the first fluorescence, and the second fluorescence so as to position the visible-light imaging sensor at the imaging position of the visible light, position the first fluorescence imaging sensor at the imaging position of the first fluorescence, and position the second fluorescence imaging sensor at the imaging position of the second fluorescence.

13. The imaging device according to claim 10, comprising

a gap that is positioned between the prism and at least one of the visible-light imaging sensor, the first fluorescence imaging sensor, and the second fluorescence imaging sensor, wherein

the gap has optical axial lengths designed according to an optical path length difference between the optical path lengths of the visible light, the first fluorescence, and the second fluorescence so as to position the visible-light imaging sensor at the imaging position of the visible light, position the first fluorescence imaging sensor at the imaging position of the first fluorescence, and position the second fluorescence imaging sensor at the imaging position of the second fluorescence.

14. The imaging device according to claim 1, wherein

the visible light includes green light, blue light, and red light,

the fluorescence includes infrared light,

the prism separates the green light, the blue light, the red light, and the infrared light included in the light from the target to be observed,

the visible-light imaging sensor includes:

a green light imaging sensor that captures the green light separated by the prism;

a blue light imaging sensor that captures the blue light separated by the prism; and

a red light imaging sensor that captures the red light separated by the prism,

the fluorescence imaging sensor includes an infrared imaging sensor that captures the infrared light separated by the prism, and

a ratio of a pixel spacing in the infrared imaging sensor to a pixel spacing in the green light imaging sensor is 1 or more and less than 5.

15. The imaging device according to claim 14, wherein

a ratio of a valid pixel area diagonal length of the infrared imaging sensor to a valid pixel area diagonal length of the green light imaging sensor is larger than 0.7 and less than 1.4.

16. The imaging device according to claim 14, wherein

light after passing through an imaging lens is input into the prism,

optical path lengths of the green light and the infrared light, from the input into the prism to imaging, are different from each other,

the green light imaging sensor is directly or indirectly fixed to the prism so as to be located at an imaging position of the green light, and

the infrared imaging sensor is directly or indirectly fixed to the prism so as to be located at an imaging position of the infrared light.

EP 4 588 417 A1

17. The imaging device according to claim 16, comprising

a long-pass filter that is provided between the prism and the infrared imaging sensor to transmit the infrared light, wherein
the long-pass filter has an optical axial length designed according to an optical path length difference between the optical path lengths of the green light and the infrared light so as to position the infrared imaging sensor at the imaging position of the infrared light.

18. The imaging device according to claim 16, wherein
the prism has optical axial lengths designed according to an optical path length difference between the optical path lengths of the green light and the infrared light so as to position the green light imaging sensor at the imaging position of the green light and position the infrared imaging sensor at the imaging position of the infrared light.

19. The imaging device according to claim 16, comprising

a gap that is positioned between the prism and at least one of the green light imaging sensor and the infrared imaging sensor, wherein
the gap has optical axial lengths designed according to an optical path length difference between the optical path lengths of the green light and the infrared light so as to position the green light imaging sensor at the imaging position of the green light and position the infrared imaging sensor at the imaging position of the infrared light.

20. A medical observation system comprising

an imaging device that captures a surgical site,
wherein
the imaging device includes:

a prism that separates visible light and fluorescence included in light from the surgical site;
a visible-light imaging sensor that captures the visible light separated by the prism; and
a fluorescence imaging sensor that captures the fluorescence separated by the prism, and
a ratio of a pixel spacing in the fluorescence imaging sensor to a pixel spacing in the visible-light imaging sensor is 1 or more and less than 5.

26

# FIG.1

# FIG.2

DICHROIC FILM 65 ———— REFLECTANCE -------- TRANSMITTANCE

# FIG.3

EP 4 588 417 A1

# FIG.4

# FIG.5

# FIG.6

# FIG.7

PIXEL AREA

VALID PIXEL AREA

9p

9p

9p

D2

# FIG.8

# FIG.9

# FIG.10

# FIG.11

# FIG.12

# FIG.13

# FIG.14

DICHROIC FILM 67 ——— REFLECTANCE ------- TRANSMITTANCE

WAVELENGTH BAND OF GREEN LIGHT VLG

TRANS-MIT-TANCE /RE-FLECT-ANCE

100%

0%

WAVELENGTH

# FIG.15

DICHROIC FILM 68 ——— REFLECTANCE ------- TRANSMITTANCE

WAVELENGTH
BAND OF BLUE
LIGHT VLB

100%

TRANS-
MIT-
TANCE
/RE-
FLECT-
ANCE

0%

WAVELENGTH

EP 4 588 417 A1

# FIG.16

REFLECTANCE ------ TRANSMITTANCE

DICHROIC FILM 69

WAVELENGTH BAND OF RED LIGHT VLR

WAVELENGTH BAND OF INFRARED LIGHT FLIR

100%

TRANS-MIT-TANCE /RE-FLECT-ANCE

0%

WAVELENGTH

EP 4 588 417 A1

# FIG.17

# FIG.18

# FIG.19

## INTERNATIONAL SEARCH REPORT

| International application No. |
| --- |
| **PCT/JP2023/032474** |

| A. | CLASSIFICATION OF SUBJECT MATTER |
| --- | --- |

*A61B 1/00*(2006.01)i; *G02B 5/04*(2006.01)i
FI:    A61B1/00 731; G02B5/04 A

According to International Patent Classification (IPC) or to both national classification and IPC

| B. | FIELDS SEARCHED |
| --- | --- |

Minimum documentation searched (classification system followed by classification symbols)

A61B1/00-1/32; G02B5/00-5/136; H04N5/222-5/257;

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | JP 2018-160800 A (JVC KENWOOD CORP.) 11 October 2018 (2018-10-11) paragraphs [0018]-[0038], fig. 8 | 1-3, 20 |
| Y | | 4-19 |
| Y | JP 2022-108769 A (TANAKA ENGINEERING INC.) 27 July 2022 (2022-07-27) paragraphs [0043], [0044], fig. 6 | 4-7, 10-13, 16-19 |
| Y | JP 2019-339 A (PANASONIC IP MANAGEMENT CORP.) 10 January 2019 (2019-01-10) paragraphs [0092]-[0098], fig. 11A, 11B | 4-7, 10-19 |
| Y | JP 2021-44790 A (MITSUI OPTICAL MANUFACTURING CO., LTD.) 18 March 2021 (2021-03-18) paragraphs [0029]-[0036], fig. 2 | 4-7, 10-13, 16-19 |
| Y | JP 2018-27272 A (SONY CORP.) 22 February 2018 (2018-02-22) paragraphs [0125]-[0144], fig. 10 | 8-13 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **20 October 2023** | **07 November 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT
### Information on patent family members

International application No.

**PCT/JP2023/032474**

| Patent document cited in search report | | | Publication date (day/month/year) | Patent family member(s) | Publication date (day/month/year) |
|---|---|---|---|---|---|
| JP | 2018-160800 | A | 11 October 2018 | US 2018/0278857 A1 paragraphs [0030]-[0050], fig. 8 | |
| JP | 2022-108769 | A | 27 July 2022 | (Family: none) | |
| JP | 2019-339 | A | 10 January 2019 | (Family: none) | |
| JP | 2021-44790 | A | 18 March 2021 | (Family: none) | |
| JP | 2018-27272 | A | 22 February 2018 | US 2019/0170647 A1 paragraphs [0138]-[0157], fig. 10 WO 2018/034075 A1 | |

Form PCT/ISA/210 (patent family annex) (January 2015)

**EP 4 588 417 A1**

## REFERENCES CITED IN THE DESCRIPTION

### Patent documents cited in the description

- JP 2017053890 A